# EUROPEAN PATENT APPLICATION

(11) **EP 2 730 653 A1**
(43) Date of publication of application: **14.05.2014**
(21) Application number: 12007554.4
(22) Date of filing: 07.11.2012
(51) Int. Cl.: C12N 15/10

(54) **Method for lysing a fixed biological sample**

(71) Applicant: QIAGEN GmbH, 40724 Hilden (DE)
(72) Inventor: The designation of the inventor has not yet been filed
(74) Representative: Roth, Carla

(57) **Abstract**

The present invention provides a method for lysing a fixed biological sample wherein the obtained lysate is suitable for being directly used in a nucleic acid analysis method, comprising:
a) contacting the fixed biological sample with an aqueous lysis composition thereby providing a lysis mixture;
b) heating the lysis mixture at ≥ 85°C to provide a lysate;

wherein inhibitors of the subsequent nucleic acid analysis method are depleted by
i) contacting the fixed biological sample in step a) with at least one compound which prevents or reduces the inhibition of the subsequent nucleic acid analysis method and/or
ii) binding inhibitors to a solid support having an anionic surface.

The method is rapid, efficient, does not require the use of chaotropic salts and does not require a prior purification of the nucleic acids prior to performing the analytical method. A portion of the obtained lysate can be used directly e.g. in an amplification reaction.

## Description

### FIELD OF THE INVENTION

The present invention provides a rapid method for releasing nucleic acids from fixed biological samples, such as FFPE samples. The obtained lysate is suitable for direct use in nucleic acid analytical methods such as nucleic acid amplification reactions.

### BACKGROUND OF THE INVENTION

If biological material, such as, for example, a tissue fragment or isolated cells, is removed from a living organism, the cells die within a short period of time. Very rapidly, the dead cells are broken down first by autolysis/fermentation and then bacterially, so that the original cell and tissue structures, components or molecules are destroyed. If cells or tissue fragments are to be removed from an organism for histological examination, it is therefore recommended to fix the biological sample taken to prevent degradation. Ideally, fixation leaves the structures of the sample substantially unchanged to allow histological assessment thereof. Fixation furthermore allows long-term preservation and archiving of the samples. For these reasons, many morphological examinations are only possible based on fixed material. If, therefore, the intention is to remove cells or tissue fragments from an organism for histological examination, it is necessary to fix the removed biological sample in order to suppress decomposition thereof.

Fixation is regularly achieved by protein-precipitating or protein-crosslinking compounds such as acids, alcohols, ketones or other organic substances such as glutaraldehyde or formaldehyde, and fixation with formaldehyde (employed e.g. in the form of a 35 percent by weight aqueous solution referred to as "formalin") followed by embedding of the fixed material in paraffin (called "formalin-fixed, paraffin-embedded" (FFPE) material) has very great importance in particular in pathology. The advantage of formalin fixation over other fixatives such as, for instance, 96 percent strength denatured alcohol is in particular that the cell and tissue structures are attained comparatively well in the fixation.

The disadvantage of fixation with cross-linking fixatives such formaldehyde is, however, in particular that it is very difficult to isolate biomolecules such as, for instance, DNA, RNA or proteins from respectively fixed material. Owing to the crosslinking effect of the used fixative such as formaldehyde, not only proteins, but also various other biomolecules including the nucleic acids present in the sample are covalently attached to one another, and as a consequence, the release and isolation of the nucleic acids (DNA or RNA) from fixed samples is very difficult. For numerous investigations on a molecular level, in particular for clinical or diagnostic applications, however, analysis of the nucleic acids is of great importance.

Therefore, numerous methods were developed for releasing and isolating nucleic acids from fixed samples.

WO 2007/068764 describes a method for isolating nucleic acids from fixed samples. The method described therein makes it possible to break the crosslinks formed by fixation in the biological sample thereby releasing nucleic acids and allowing to isolate one type of nucleic acid, that is either DNA or RNA, which may then be followed, for example, by PCR or RT-PCR analysis.

WO2005/075642 describes a method for simultaneous extraction of DNA and RNA from a biological sample, including FFPE samples. The FFPE sample is deparaffinised and digested using a lysis buffer comprising a chaotropic agent, an ionic detergent and a proteolytic enzyme. The sample is digested for at least 5, preferably 10 hours, to release the RNA and DNA, phenol-chloroform is added and the phases are separated. The aqueous phase comprises mainly RNA, the organic phase mainly DNA. RNA can then be recovered from the aqueous phase using alcohol precipitation. The DNA is recovered from the organic phase. This method inter alia has the drawback that it is time consuming and requires the use of toxic agents and furthermore, the use of costly enzymes.

WO 2011/104027 describes a method suitable for isolating DNA and RNA in parallel from a fixed sample. For lysis of the sample, a proteolytic enzyme is used. This has the drawback that the method is time consuming and furthermore requires the use of costly enzymes.

WO 01/46402 proposes heating the FFPE material with a chaotropic solution comprising an effective amount of a guanidinium compound at a temperature of from 75 to 100 degrees C. for from 5 to 120 minutes. The disadvantage of this method is, however, that the conditions under which the material is heated often leads to an at least partial destruction of the biomolecules, especially sensitive biomolecules such as, for instance, RNA. Moreover, the treatment times necessary for sufficient disconnection of the formaldehyde crosslinking are often very long.

US 2005/0014203 proposes initially heating a fixed biological sample in order to disconnect the crosslinking of the biomolecules at least partly, and subsequently to incubate the sample treated in this way with a proteolytic enzyme, for example with proteinase K, in order to decompose the tissue and the cellular structures of the tissue. The disadvantage of this method is likewise that it is time consuming and requires the use of costly enzymes.

The prior art methods for releasing nucleic acids from fixed samples inter alia have the drawback that they are time consuming and furthermore, often require the use of toxic and/or costly reagents. Furthermore, most prior art methods require a purification of the nucleic acids from the fixed samples in order to allow an analysis of the nucleic acids e.g. by using amplification based methods. This has the disadvantage that this prolongs the preparation time. Furthermore, purifying the nucleic acids is also challenging because nucleic acids comprised in fixed samples are often of poor quality because they are fragmented and/or degraded. Thus, nucleic acids may be lost during the purification process. Furthermore, the prior art methods have the disadvantage that they are generally laborious and require multiple steps involving different reagents. Therefore, it is difficult to automate these methods.

It is the object of the present invention to overcome at least one drawback of the prior art methods. In particular, it is an object of the present invention to provide a rapid method which renders nucleic acids comprised in fixed biological samples accessible for analytical methods such as in particular amplification based analytical methods. In particular, it is one object of the present invention to provide a rapid method which does not require a purification of the nucleic acids from fixed samples prior to analysis. Furthermore, it is one object to provide a rapid method which does not require or at least reduces the use of toxic chemicals. Furthermore, it was one object to provide a method that is suitable for automation.

### SUMMARY OF THE INVENTION

The present invention provides a rapid and efficient method for releasing nucleic acids from fixed biological samples. The method does not require the use of chaotropic agents or proteolytic enzymes. Furthermore, it is not necessary to purify the nucleic acids prior to analysis, because the present invention renders a lysate that can be directly used in nucleic acid analysis methods, including amplification based analytical methods. The method is fast, reliable, automatable and thus particularly suitable for high throughput applications.

According to a first aspect, a method is provided for lysing a fixed biological sample wherein the obtained lysate is suitable for being directly used in a nucleic acid analysis method, comprising:
a) contacting the fixed biological sample with an aqueous lysis composition thereby providing a lysis mixture;
b) heating the lysis mixture at ≥ 85°C to provide a lysate;
wherein inhibitors of the subsequent nucleic acid analysis method are depleted by
i) contacting the fixed biological sample in step a) with at least one compound which prevents or reduces the inhibition of the subsequent nucleic acid analysis method and/or
ii) binding inhibitors to a solid support which preferably comprises an anionic surface.

According to a second aspect, a nucleic acid analysis method is provided, comprising
a) lysing a fixed biological sample according to the method of the first aspect, and
b) using at least a portion of the obtained lysate in a nucleic acid analysis method.

Other objects, features, advantages and aspects of the present application will become apparent to those skilled in the art from the following description and appended claims. It should be understood, however, that the following description, appended claims, and specific examples, while indicating preferred embodiments of the application, are given by way of illustration only. Various changes and modifications within the spirit and scope of the disclosed invention will become readily apparent to those skilled in the art from reading the following.

### DETAILED DESCRIPTION OF THE INVENTION

According to a first aspect, a method is provided for lysing a fixed biological sample wherein the obtained lysate is suitable for being directly used in a nucleic acid analysis method, comprising:
a) contacting the fixed biological sample with an aqueous lysis composition thereby providing a lysis mixture;
b) heating the lysis mixture at ≥ 85°C to provide a lysate;
wherein inhibitors of the subsequent nucleic acid analysis method are depleted by
i) contacting the fixed biological sample in step a) with at least one compound which prevents or reduces the inhibition of the subsequent nucleic acid analysis method and/or
ii) binding inhibitors to a solid support which preferably comprises an anionic surface.

The method according to the present invention allows to efficiently lyse fixed biological samples thereby releasing comprised nucleic acids and rendering them accessible for analytical methods. The method is rapid, automatable and provides a lysate that can be directly used in nucleic acid analysis methods, such as amplification based analytical methods. Thus, a prior purification of the nucleic acids is obsolete when using the method according to the present invention. Hence, "directly" in this respect means that it is not necessary to isolate and in particular to purify the nucleic acids comprised in the lysate prior to performing the subsequent nucleic acid analysis method which preferably is an amplification method. Therefore, no nucleic acid isolation or purification is performed prior to performing the nucleic acid analysis method. However, as described below it is within the scope of the present invention to further process the lysate prior to subjecting an aliquot thereof to the nucleic acid analysis method. E.g. the lysate may be cleared and an aliquot of the cleared lysate is then used in the nucleic acid analysis method which preferably is an amplification reaction. Furthermore, the obtained lysate may also be treated with enzymes such as RNase, DNase or reverse transcriptase prior to performing the nucleic acid analysis method.

The present invention enables high throughput processing of large amounts of fixed samples such as e.g. FFPE samples present in biobanks or other clinical settings.

The individual steps of the method as well as preferred embodiments thereof will now be described in detail.

### STEP A

In step a) the fixed biological sample is contacted with an aqueous lysis composition thereby providing a lysis mixture. Said lysis composition, which preferably is a lysis solution, comprises at least one reagent that promotes the lysis of the biological sample. Preferably, the aqueous lysis composition comprises a detergent for said purpose, in particular a non-ionic detergent. Suitable embodiments are described subsequently. Depending on the composition of the lysis solution, lysis may be directly initiated. Lysis is promoted and hence assisted by heating in step b).

As the present method provides a method which is directly suitable for use in a nucleic acid analysis method such as e.g. an amplification reaction, it is essential to deplete inhibitors of the subsequent nucleic acid analysis method that are released from the fixed biological sample during lysis. E.g. typical inhibitors of a nucleic acid amplification reaction which usually originate from the biological sample and are released during lysis include but are not limited to proteoglycans, proteins and sugars. It is important to ensure that respective inhibitors do not disturb the subsequent analytical method such as e.g. the amplification. In the lysis method according to the present invention respective inhibitors are depleted by
i) contacting the fixed biological sample in step a) with at least one compound which prevents or reduces the inhibition of the subsequent nucleic acid analysis method and/or
ii) binding inhibitors to a solid support which preferably comprises an anionic surface.

As is shown by the examples, both measures alone are sufficient to deplete inhibitors such as in particular PCR inhibitors from the lysate. However, a combination of both measures is preferred. The term "depletion" of inhibitors and similar terms used herein are used in a broad sense and do not include a limitation regarding the mode of action. In particular, said terms include e.g. unspecific complexing of inhibitors by the used compounds, the binding of the inhibitors to the surface of the solid support and the like. The use of the polymer and/or the solid support preferably having an anionic surface as described herein basically inhibits the inhibitory action of inhibitors that are released during lysis of the fixed biological sample. An inhibitor is "depleted" in the sense of the present invention, if the subsequent analysis method such as e.g. an amplification reaction shows an improved performance due to the addition of the compound and/or the use of the solid support during lysis compared to when said polymer is not added and/or no respective solid support is used during lysis to deplete inhibitors comprised in the lysate.

The individual measures that can be used either alone or in combination in order to deplete inhibitors that are released during lysis are subsequently described in detail.

### Compound which prevents or reduces the inhibition of the subsequent nucleic acid analysis method

According to one embodiment, the fixed biological sample is contacted in step a) with at least one compound which prevents or reduces the inhibition of the subsequent nucleic acid analysis method, which preferably is an amplification based analytical method. Thus, the addition of the compound has the effect that the subsequent analysis method such as e.g. an amplification reaction shows an improved performance when said compound is added in step a) compared to when said compound is not added. It is assumed that respective compounds such as the polymers described subsequently prevent or reduce the inhibition by unspecifically binding or complexing potential inhibitors which are thereby "inactivated". Which compounds act as inhibitors also depends on the analytical method that is subsequently performed. E.g. typical inhibitors of a nucleic acid amplification reaction which usually originate from the sample and are released during lysis include but are not limited to proteoglycans, proteins and sugars. The compound which prevents or reduces the inhibition of the subsequent nucleic acid analysis method may be conveniently incorporated into the aqueous lysis composition. Said compound which preferably is a polymer may be comprised in the aqueous lysis composition in a concentration that is selected from 0.01% (w/v) to 2% (w/v), 0.015 % (w/v) to 1.5 % (w/v), 0.02 % (w/v) to 0.1 % (w/v), 0.02% (w/v) to 0.5% (w/v), 0.025% (w/v) to 0.3% (w/v), 0.035% (w/v) to 0.2% (w/v) and 0.04% (w/v) to 0.15% (w/v). The suitable concentration of said compound also depends e.g. on the processed fixed sample material and the amount of inhibitors that are released during lysis. Suitable concentrations can be determined for individual embodiments of the compound that is used for inhibitor depletion and fixed samples to be processed following the teachings described herein using routine experiments.

According to one embodiment, the compound which prevents or reduces the inhibition of the subsequent nucleic acid analysis method is a polymer. Preferably, the polymer is water-soluble and is selected from the group of binders, thickeners and water-soluble polyanionic polymers which preferably comprise carboxylate groups. Suitable and preferred embodiments are described below. Also, a mixture of respective polymers can be used. Details and preferred embodiments are described in the subsequent section.

### Water-soluble polyanionic polymer

According to one embodiment, the compound which is used to prevent or reduce an inhibition of the subsequent analytical method is a water-soluble polyanionic polymer comprising carboxylate groups, such as e.g. polyacrylic acid (poly(acrylic acid) or PAA). As shown in the examples, using a respective lysing procedure wherein a respective water-soluble polyanionic polymer comprising carboxylate groups is added in step a) provides a lysate that can be used directly in an amplification reaction. Inhibitors of amplification reactions are advantageously depleted due to the addition of the water-soluble polyanionic polymer, it is assumed by way of unspecific complex formation. As is shown by the examples, said water-soluble polyanionic polymer is decisive for reducing the inhibiting effect of amplification inhibitors that are comprised in the lysed sample. Excellent results are achieved even if said polymer such as PAA is used alone in order to deplete inhibitors. As described above, respective inhibitors are e.g. released from the fixed biological sample during lysis. Thus, the incorporation of the water-soluble polyanionic polymer comprising carboxylate groups during lysis has the effect that the subsequent analysis method such as e.g. an amplification reaction shows an improved performance if said water-soluble polyanionic polymer comprising carboxylate groups is used during lysis compared to if said polymer is not being used during lysis. That the amplification performance can be improved was particularly surprising because water-soluble polyanionic polymers such as e.g. polyacrylic acid are in fact known to act as PCR inhibitors and thus were known to have the opposite effect. Hence, that respective water-soluble polyanionic polymers comprising carboxylate groups that are known to be amplification inhibitors themselves, such as polyacrylic acid, can be used to reduce the inhibitory effect of PCR inhibitors thereby improving the performance of the amplification reaction was highly unexpected. Without wishing to be bound by theory, it is believed that the inhibitory action of respective water-soluble polyanionic polymers such as PAA on the amplification reaction is somehow "neutralized" or otherwise overcome by the amplification inhibitors that are released from the fixed biological sample during lysis. It is believed that respective water-soluble polyanionic polymers form complexes with the inhibitors that are released from the fixed biological sample, wherein said complex formation somehow eliminates or reduces the inhibitory action of both inhibitors (of the water-soluble polyanionic polymer and of the inhibitors released from the biological sample). Furthermore, as shown by the examples, using a water-soluble polyanionic polymer comprising carboxylate groups for neutralizing amplification inhibitors provides results that are improved compared to using other polymers such as polyvinylpyrrolidone (PVP).

When following the teachings of the present invention and adding a water-soluble polyanionic polymer comprising carboxylate groups during lysis, a lysate is provided that can be used directly in an analytical method such as e.g. a nucleic acid amplification reaction. In fact, the obtained lysate can even be added in rather large amounts into the amplification reaction without significant inhibition of the subsequent analysis method. That a rather large amount of lysed sample can be transferred into or used in the analysis method is a particular advantage, as thereby the sensitivity of the subsequent analytical method can be improved because e.g. more nucleic acids are transferred with the lysate into the analytical method.

This is a particular advantage when preparing a lysate from a fixed biological sample, because nucleic acids comprised therein are often degraded.

Preference within the scope of the invention is given to water-soluble polyanionic polymers, including copolymers, comprising carboxylate-containing monomers, such as preferably comprising acrylic acid, methacrylic acid and/or maleic acid. According to one embodiment, said polymer comprises only two types of monomers. Preferably, only one type of monomer is comprised in the water-soluble polyanionic polymer.

In a preferred embodiment, the polymer is selected from polyacrylic acid, polymaleic acid and poly(acrylic acid-co-maleic acid). Most preferably, polyacrylic acid is used. As shown in the examples, polyacrylic acid is very efficient in preventing the inhibition of amplification reactions and thus in depleting inhibitors that are released during lysis of the fixed sample.

If a copolymer is used, it may additionally comprise polar monomers which do not contain carboxylate. Examples of respective polar monomers encompass lactide and/or vinylpyrrolidone. According to one embodiment, said polymer comprises only type of the respective polar monomers. According to one embodiment the copolymer is poly(acrylic acid-co-lactide), poly(acrylic acid-co-vinylpyrrolidone), poly(maleic acid-co-lactide), poly(maleic acid-co-vinylpyrrolidone), poly(acrylic acid-co-maleic acid-co-lactide) or poly(acrylic acid-co-maleic acid-co-vinylpyrrolidone). Furthermore, the copolymer additionally may comprise anionic monomers not containing carboxylate. According to one embodiment, the copolymer is a block copolymer.

Also mixtures of water-soluble polyanionic polymers comprising carboxylate groups can be used in step a).

The molecular weight of the water-soluble polyanionic polymer is preferably chosen such that it is present in a solved form in the lysis mixture and preferably also in the aqueous lysis composition if comprised therein.

The average molecular weight of the at least one water-soluble polyanionic polymer is preferably selected from a range of 2,000 Da to 500,000 Da, 5,000 Da to 475,000 Da, 10,000 to 450,000 Da, 25,000 to 400,000 Da, 35,000 to 350,000 Da, 50,000 Da to 300,000 Da, 75,000 to 300,000 Da, 100,000 Da to 300.000 Da, 150,000 to 300,000 Da and 200,000 Da to 275,000 Da. In a preferred embodiment the average molecular weight is approx. 250,000 Da.

As described above, the water-soluble polyanionic polymer or mixture of water-soluble polyanionic polymers may be comprised in the aqueous lysis composition in a concentration that is selected from 0.01% (w/v) to 1% (w/v), 0.02% (w/v) to 0.5% (w/v), 0.025% (w/v) to 0.3% (w/v), 0.035% (w/v) to 0.2% (w/v) and 0.04% (w/v) to 0.15% (w/v). Preferably, it is comprised in the aqueous lysis composition that is added to the sample in a concentration selected from 0.025% (w/v) to 0.15% (w/v), 0.03% (w/v) to 0.1% (w/v), 0.03% (w/v) to 0.075% (w/v) and 0.035% (w/v) to 0.05% (w/v). Respective concentrations are particularly suitable when using polyacrylic acid as polymer. However, also higher concentrations may be feasible, e.g. if processing large, cell-rich fixed sample materials. Care should be taken that the polymer such as PAA is not comprised in the lysate in a concentration wherein it acts itself as inhibitor. As described above, the suitable concentration to be used may also depend on the amount of released inhibitors and thus the type or amount of fixed sample to be processed. Suitable concentrations can be determined by the skilled person for specific sample materials and specific water-soluble polyanionic polymers.

### Binders and/or thickeners

According to one embodiment, the compound which is used to prevent or reduce an inhibition of the subsequent analytical method is a polymer which acts as a binder and/or thickener. Said binder and/or thickener is preferably water-soluble and is incorporated into the aqueous lysis composition. It may be selected from the group consisting of polyvinylpyrrolidone (PVP), polyoxazoline, polyethylene glycol, polyvinyl alcohol and Luvitec.

Said polymer can be comprised in the aqueous lysis composition in a concentration that is selected from 0.01% (w/v) to 1% (w/v), 0.02% (w/v) to 0.5% (w/v), 0.025% (w/v) to 0.3% (w/v), 0.035% (w/v) to 0.2% (w/v) and 0.04% (w/v) to 0.15% (w/v). Preferably, it is comprised therein in a concentration selected from 0.05% (w/v) to 0.15% (w/v) and 0.075% (w/v) to 0.125% (w/v). According to one embodiment, polyvinylpyrrolidone is used as polymer and preferably, is incorporated into the aqueous lysis composition in said concentrations. Suitable binders and/or thickeners that can be used for depleting inhibitors are also described in WO 2010/003493. As is shown in the examples, such binders and/or thickeners such as PVP are efficient in preventing the inhibition of amplification reactions when used in combination with a solid support having a polyanionic surface that binds inhibitors such as e.g. a solid support carrying carboxyl groups at their surface.

### Further examples

According to one embodiment, the compound which is used to prevent or reduce an inhibition of the subsequent analytical method comprises at least one anionic group. Said at least one anionic group may be selected from the group consisting of carboxyl, sulfonate, sulphate, phosphonate and phosphate groups. Preferably, said compound comprises at least one carboxyl and/or sulfonate group. It may be a betaine or sulfobetaine. Suitable examples of such compounds such as the water-soluble polyanionic polymers comprising carboxylate-containing monomers were described above.

According to one embodiment, the compound which is used to prevent or reduce an inhibition of the subsequent analytical method such as an amplification reaction is an anionic or zwitterionic detergent. It may be comprised in the lysis composition. Suitable concentrations may be selected from 0.01% (w/v) to 5% (w/v), 0.05% (w/v) to 2% (w/v), 0.075% (w/v) to 1% (w/v) and 0.1% (w/v) to 0.5% (w/v). However, as described above, depending on the used sample material and the amount of inhibitors that are released during lysis, also higher concentrations can be used. Suitable concentrations can be determined using routine tests. Using a respective detergent is advantageous, because said detergent also assists the lysis of the biological sample material. Preferably, said detergent comprises a sulfonate group. According to one embodiment, it is a betaine, preferably a sulfobetaine. It may be a tetradecyl derivative. According to one embodiment, the lysis composition comprises 3-(N,N-Dimethylmyristylammonio)propanesulfonate (Sulfobetain 3-14; SB3-14; CAS No. 14933-09-6). Said compound efficiently prevents or reduces an inhibition of amplification reactions A respective detergent can also be used in combination with another compound which prevents or reduces an inhibition of the subsequent analytical method such as e.g. in combination with the water-soluble polyanionic polymer described above.

Other additives that can be additionally used to deplete inhibitors released from the fixed biological sample include, but are not limited to, ion-exchange materials or non-ionic resins that adsorb ionic species through hydrophobic and polar interactions such as e.g. Amberlite XAD-7.

### Detergent

According to a preferred embodiment, the aqueous lysis composition comprises a detergent. Detergents support the lysis of the sample and dissolve protein aggregates. Also a mixture of detergents can be used.

Preferably, a non-ionic detergent or a combination of non-ionic detergents is comprised in the aqueous lysis composition. Preferably, the aqueous lysis composition and also the lysis mixture comprises only non-ionic detergent(s). Substances which are suitable for this purpose are, in principle, any non-ionic surfactant which has no adverse effect on the nucleic acids to be examined and allows performing the subsequent nucleic acid analysis method with the obtained lysate. The non-ionic surfactant may be selected from the group consisting of polyoxyethylene fatty alcohol ethers, polyoxyethylene alkylphenyl ethers, polyoxyethylene-polyoxypropylene block copolymers, polysorbates and alkylphenol ethoxylates, preferably polyoxyethylene fatty alcohol ethers, polysorbates and/or polyoxyethylene alkyl-phenyl ethers.

The term "fatty alcohol" in particular means for the purposes of the present invention alcohols having a chain length of from 6 to 22 carbon atoms, preferably 8 to 20 carbon atoms, preferentially 10 to 18 carbon atoms, particularly preferably 12 to 18 carbon atoms. Preference is in particular given to alcohols having 12, 14, 16 or 18 carbon atoms. Although the fatty alcohols may be mono- or polyunsaturated, they are preferably saturated fatty alcohols. The term "polyoxyethylene" in particular means for the purposes of the present invention an HO-(CH2CH2O)n unit, with n being preferably an integer from 2 to 150, further preferably from 4 to 120, still further preferably from 8 to 80, and most preferably an integer selected from 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 and 50.

Preferred examples of suitable polyoxyethylene fatty alcohol ethers are polyethoxylated lauryl, cetyl, oleyl, or stearyl alcohols which may be used alone or as mixture. According to a preferred embodiment of the invention, the at least one polyoxyethylene fatty alcohol ether comprises a fatty alcohol moiety having from 6 to 22 carbon atoms and a polyoxyethylene moiety having from 2 to 150 (CH₂CH₂O) units. Preferably, the polyoxyethylene fatty alcohol ether is selected from the group consisting of polyoxyethylene lauryl ether, polyoxyethylene cetyl ether (Brij-58), polyoxyethylene stearyl ether and/or polyoxyethylene oleyl ether.

As alkylglucoside, preferably a non-ionic surfactant from the group of the polysorbates, preferably polysorbate 20 (Tween 20), polysorbate 40 or polysorbate 80, more preferred polysorbate 20 is used.

Preferred examples of polyoxyethylene alkyl phenyl ethers include nonylphenol ethoxylates (Tergitol), polyethylene glycol p-(1,1,3,3-tetramethylbutyl)-phenyl ether (Triton X-100) and nonylphenylpolyethylenglycol (Nonidet P-40).

According to one embodiment, at least two non-ionic detergents are used in combination. Preferably, an alkylglucoside, preferably a polysorbate, and a polyoxyethylene alkyl phenyl ether are used in combination.

According to one embodiment, the non-ionic detergent is selected from the group consisting of polysorbate 20 (Tween 20), Tergitol, Triton X-100, Brij-58 and Nonidet P-40. Preferably, at least two respective non-ionic detergents are comprised in the aqueous lysis composition. The concentration of the non-ionic detergent or the combination of non-ionic detergents in a respective lysis composition is preferably selected from 0.05% (v/v) to 5% (v/v), 0.1 % (v/v) to 3% (v/v), 0.15% (v/v) to 2.5% (v/v), 0.2% (v/v) to 2% (v/v), 0.3% (v/v) to 1.5% (v/v) and 0.4% (v/v) to 1% (v/v). Preferably, Tween 20 and/or Nonidet P-40, preferably a combination of both, are comprised in the aqueous lysis composition.

Preferably, the overall concentration of non-ionic detergent(s) in the lysis mixture is ≤ 5%, preferably ≤ 3% (v/v), more preferably ≤ 2% (v/v).

### Buffering agent

According to one embodiment, the fixed biological sample is contacted in step a) with at least one buffering agent. Preferably, the buffering agent is comprised in the aqueous lysis composition. The buffering agent should be capable of buffering the aqueous lysis composition and preferably also the obtained lysate at a pH range that is compatible with the pH range that is required for performing the subsequent analytical method, such as e.g. an amplification reaction. This, as the pH of the lysate is strongly affected by the aqueous composition that is used for lysis and the lysate is directly used in the subsequent analytical method. Preferably, a buffering agent is used in the aqueous lysis composition so that the pH of the composition is in a range selected from 7 to 11, 7.5 to 10, 7.75 to 9.5 and 8.0 to 9. Furthermore, preferably, also the obtained lysate has a pH value that lies in said range. Respectively buffered aqueous lysis compositions are particularly suitable for providing a lysate that is suitable for performing a nucleic acid amplification reaction. At such pH values, carboxyl groups of the water-soluble polyanionic polymer are also present as carboxylate groups.

The buffering agent can be comprised in the aqueous lysis composition in a concentration of 5mM to 100mM, preferably 7.5mM to 80mM, more preferably 10-50 mM. Preferably, the buffer substance is selected from the group consisting of Tris, MOPS, HEPES, phosphate and borate, more preferred selected from Tris and borate. As is shown by the examples, using borate as buffering agent is particularly preferred because the results in an amplification reaction are improved.

### Chelating agent

According to one embodiment, the fixed biological sample is contacted in step a) also with at least one chelating agent, which preferably is suitable for chelating divalent cations. Suitable chelating agents include but are not limited to diethylenetriaminepentaacetic acid (DTPA), ethylenedinitrilotetraacetic acid (EDTA), ethylene glycol tetraacetic acid (EGTA) and N,N-bis(carboxymethyl)glycine (NTA). According to a preferred embodiment, EDTA is used. As used herein, the term "EDTA" indicates *inter alia* the EDTA portion of an EDTA compound such as, for example, K₂EDTA, K₃EDTA or Na₂EDTA. Using a chelating agent such as EDTA has the advantageous effect that nucleases such as DNases and RNases are inhibited. According to one embodiment, the chelating agent is comprised in the aqueous lysis composition. The chelating agent is comprised in the resulting lysate in a concentration, wherein it does not inhibit the subsequent downstream reaction, e.g. an amplification reaction. The chelating agent preferably is employed in a concentration of 0,5mM to 5mM, preferably 0.75mM to 2mM, most preferred 1mM to 1.5mM in the aqueous lysis composition. Preferably, EDTA is used.

### Further additives

Further additives can be incorporated into the lysis mixture and/or the aqueous lysis composition that protect the released nucleic acids from degradation. E.g. suitable DNase and/or RNase inhibitors can be added. The incorporation of RNase inhibitors is particularly recommended if RNA is the nucleic acid of interest as RNA is very susceptible to degradation. Suitable protective agents are known in the prior art and, thus, do not need any detailed description here. As the lysed sample is directly subjected to the analytical method such as e.g. an amplification reaction without prior purification of the nucleic acids from the lysate, it is important to ensure that the performed lysis does not introduce one or more inhibitors of the subsequent analysis method in a concentration that would inhibit the performance of the subsequent analytical method to an extent that the analytical method cannot be adequately performed. The tolerable concentration depends on the analytical method to be performed, e.g. the used enzymes and their susceptibility to inhibitors and the sensitivity that is required for the adequate performance of the analytical methods. These parameters can also vary from sample to sample and intended analytical method.

### Preferred aqueous lysis compositions

A particularly preferred aqueous lysis composition comprises or consists of
- at least one compound which prevents or reduces the inhibition of the subsequent analytical method, preferably a polymer, more preferred a water-soluble polyanionic polymer as described above, preferably polyacrylic acid, and/or PVP, in a concentration of 0.01% (w/v) to 0.5% (w/v), preferably 0.02% (w/v) to 0.2% (w/v), more preferred 0.03% (w/v) to 0.15% (w/v);
- at least one, preferably at least two non-ionic detergents, preferably Polysorbate 20 (Tween 20) and nonylphenylpolyethylenglycol (Nonidet P40), wherein, preferably, each non-ionic detergent is comprised in a concentration of 0.2% (v/v) to 0.6% (v/v), preferably 0.4% (v/v) to 0.5% (v/v);
- a buffering agent, preferably TRIS or borate,
- optionally a chelating agent in a concentration ≤ 2mM, preferably ≤ 1.5mM.

The pH value of the respective aqueous lysis composition preferably lies in a range of 8.0 to 9.5, preferably 8.5 to 9.

The described aqueous lysis composition is in particular compatible for providing a lysate that is suitable for direct use in an amplification reaction such as a PCR. As is shown in the examples, a respective aqueous lysis composition achieves in combination with heating step b) an efficient lysis of the fixed biological sample and furthermore, does not comprise additives in a concentration that would substantially inhibit a respective amplification reaction. Therefore, lysates are provided which can be directly used in a nucleic acid analysis methods such as amplification based analytical methods.

### STEP B)

In step b), the lysis mixture that was obtained in step a) is heated at ≥ 85°C to provide a lysate. This heating step is important in order to lyse the fixed biological sample and ensure an efficient release of the comprised nucleic acids. Furthermore, performing a respective heating step during lysis has the advantage that nucleases comprised in the lysate are denatured and thereby are inactivated. The necessary heating incubation time also depends on the type of fixation, the degree of fixation, the amount of fixed sample to be lysed and the incubation temperature. Preferably, the lysis mixture is heated at ≥ 90°C, preferably ≥ 95°C. Such a heating step is also referred to as boiling lysis. Preferably, in step b), the lysis mixture is heated for at least 5min, at least 7.5min, at least 10min, at least 12.5min, at least 15min, at least 17.5 min, at least 20min, at least 22.5min, at least 25min, at least 27.5min or at least 30min. Suitable heating periods may be selected from 5min to 45min, 7.5min to 42.5min, 10min to 40min, 12.5min to 37.5min and 15min to 35min. As is shown in the examples, the method according to the present invention allows to lyse the fixed biological sample and release the comprised nucleic acids from different fixed samples thereby rendering them accessible to analytical methods such as reverse transcription and/or amplification. Furthermore, as it is ensured that potential inhibitors of the analytical method are efficiently depleted, the obtained lysate can be directly used in the nucleic acid analysis method such as e.g. an amplification reaction.

Preferably, after the heat incubation step, the obtained lysate is mixed, e. g. vortexed or otherwise strongly agitated or shaken. Furthermore, the heated sample is preferably allowed to cool down. It may be cooled at room temperature or below. Preferably, it is allowed to cool down to a temperature below 50°C, more preferably it is cooled down at least down to room temperature. Such a cooling step is advantageous in particular when processing embedded fixed samples such as paraffin embedded fixed samples because the embedding material solidifies during cooling thereby facilitating preventing a carry-over of embedding material. Furthermore, it was found that a respective cooling step promotes the formation of precipitates which comprise, respectively, constitute themselves inhibitors or contaminants of the subsequent analytical method. The efficient precipitate formation allows to efficiently remove respective precipitates e.g. by performing a clearing step as is described below, thereby providing a lysate from which inhibitors and contaminants such as precipitates are efficiently depleted. Suitable and preferred embodiments of a respective clearing step which is optional but preferred though, will also be described subsequently.

### Binding of inhibitors to a solid support

According to one embodiment, inhibitors of the subsequent nucleic acid analysis method such as e.g. an amplification method are depleted by binding inhibitors to a solid support which preferably has an anionic surface. As described above, respective inhibitors in particular originate from the fixed sample during lysis. As is shown in the examples, using a solid support comprising an anionic surface which can bind inhibitors such as e.g. a carboxylated surface during lysis of the fixed sample renders a lysate that can be directly used in a nucleic acid amplification method even if no polymer is additionally used in order to deplete inhibitors. However, preferably, said solid support having an anionic surface is used in combination with a polymer which prevents or reduces the inhibition of the subsequent nucleic acid analysis method as the analytical results may be improved as is shown by the examples.

The solid support comprises an anionic surface which binds to inhibitors that are released during lysis. The term "binding" is used in a broad sense and refers to any interaction of the inhibitors with the solid support. Binding preferably is achieved by adsorption. By binding the inhibitors to the solid support, they are depleted from the lysate and the subsequent analytical method such as an amplification can be performed directly using the obtained lysate without having to purify the nucleic acids in advance. It is preferred to separate the solid support with the bound inhibitors from the lysate. Thereby, it is ensured that the inhibitors are not again released during the subsequent handling and/or during the performance of the subsequent nucleic acid analysis method.

Using a respective solid support having an anionic surface for binding respective inhibitors, such as in particular amplification inhibitors, is particular advantageous as it allows to also clear the lysate at the same time. It was found that anionic surfaces which bind to inhibitors such as in particular PCR inhibitors also bind precipitates that are formed during the lysis of the fixed sample. Thus, respective precipitates which may also comprise or consist of inhibitors can also be removed when using a respective solid support. Further details regarding the lysate clearing are also described subsequently. It is preferred to use a respective solid support having an anionic surface even if a polymer such as e.g. polyacrylic acid is used which alone is sufficient in order to deplete inhibitors from the lysate because the solid support having an anionic surface does not only deplete inhibitors, but also binds to and thus allows to remove precipitates that are formed during lysis thereby allowing to clear the lysate. Furthermore, a respective combinatory use may also allow to use lower concentrations of the respective polymer, while ensuring an efficient depletion.

The term "surface" as used herein in particular refers to a portion of a solid support which comes into contact with a liquid when the solid support is contacted therewith. The solid support comprises anionic groups and thus provides an anionic surface that allows to bind inhibitors, in particular PCR inhibitors. For this purpose, it is also within the scope of the present invention to functionalize the surface of a solid support with appropriate functional groups. Suitable examples will be described below.

It is preferred that the anionic surface comprises multiple anionic groups such as acidic functional groups and thus provides a polyanionic surface. E.g. the solid support and/or its surface may comprise or consist of a polymer capable of forming a polyanionic structure. E.g. the polyanionic polymer, which may also be a copolymer or terpolymer, may be a polycarboxylate or a carboxylated polymer or a polyester capable of forming a polyanionic structure. According to one embodiment, said polymer is a carboxylated polymer e.g. based on vinyl methyl ether, maleic anhydride, styrene, linear or branched alkenes or acrylic acid and its derivatives. For example, polymers based on styrene, vinyl methyl ether, linear or branched alkenes-such as e.g. 1-octadecene or isopropene and maleic acid or acrylic acid, wherein the carboxyl functionalities can be optionally esterified to different degrees, can be employed. Preferred exemplary acrylic acid alkyl esters include the following: methyl acrylate, ethyl acrylate, vinyl acrylate, propyl acrylate, butyl acrylate, hexyl acrylate, octyl acrylate, decyl acrylate, dodecyl acrylate, myristyl acrylate, lauryl acrylate, cetyl acrylate, stearyl acrylate, methyl methacrylate, ethyl methacrylate, propyl methacrylate, butyl methacrylate, isobutyl methacrylate, hexyl methacrylate, 2-ethylhexyl acrylate, 2-ethylhexyl methacrylate, phenyl methacrylate, octyl methacrylate, decyl methacrylate, dodecyl methacrylate, myristyl methacrylate, lauryl methacrylate, cetyl methacrylate, where the acrylic acid methyl ester is particularly preferred. Polyesters that are capable of forming a polyanionic structure illustrate further suitable polymers. In general, the monomers themselves can possess groups that after polymerisation constitute or form anionic sites. Moreover, the polyesters can also be subsequently provided with groups forming anions, e.g. through unsaturated sites or other functional groups present in the polyester. Dicarboxylic acids, such as succinic acid, adipic acid, phthalic acid or maleic acid, and as alcohols, tartaric acid or other dihydric or trihydric alcohols that comprise at least one additional anionic functionality, are preferably used as the monomers. When maleic acid is used as the monomer, dihydric or trihydric alcohols-without additional anionic functionalities-can be used for the polymerisation, when the residual double bond of the maleic acid in the polymer is utilized, for example, to subsequently introduce anion forming groups, such as for example acrylic acid or methacrylic acid. In principle, all anionic structures formed from polymers are suitable to provide the surface of the solid support, with carboxylated polymers being preferred. Respective polymers may also form a coating on a solid support, thereby providing an appropriate polyanionic surface for binding inhibitors. Furthermore, the solid support may comprise iminodiacetic acid groups.

According to one embodiment, the surface of the solid support comprises acidic functional groups. According to one embodiment, the pKa value of said functional groups lies in a range of 0 to 7, preferably 1 to 6, more preferred 1 to 5. Suitable examples include acidic groups such as carboxyl groups. Further suitable functional groups include betains, sulfonate, phosphonate, phenol and phosphate groups. Also mixtures of respective functional groups can be present on the surface. Preferably, the surface comprises carboxyl groups as functional groups, as a respective polyanionic surface is highly efficient in binding and thus depleting inhibitors such as amplification inhibitors and additionally binds precipitates that are formed during lysis thereby additionally providing a lysate clearing effect.

The manufacture of carboxylated polymers is well known from the prior art as these polymers are employed in a great number of other technical applications, they are for the most part commercially available. Furthermore, it is also well-known how to provide and e.g. functionalize a solid support with a respective polyanionic surface, preferably a carboxylated surface. Examples include, but are not limited to, coatings, the deposition of a film or layer and the manufacture of the solid support by polyanionic polymers.

As described above, the anionic functional groups can be provided by the surface material itself, e.g. acidic groups such as carboxyl groups (e.g. if the surface is made of a polyacrylate) or respective functional groups can be provided by ligands or polymers that are attached to or are provided covalently or non-covalently to the surface of the solid support.

Suitable solid supports may be made of or may comprise in particular at their surface a material selected from the following group
- a material comprising or consisting of silicon such as silica and polysilicic acid materials, quartz, borosilicates, silicates, diatomaceous earth or glasses,
- a material comprising or consisting of an organic or inorganic polymer such as poly(meth)acrylate, polyurethane, polystyrene, polystyrol, polyacrylamide, a divinylbenzene polymer, a styrene divinylbenzene polymer, polyethylene, polypropylene, polyvinylidene fluoride, polyacrylonitrile, polyvinylchloride, polyacrylate, polyacrylamide, polymethacrylate or a methyl methacrylate polymer;
- a material comprising or consisting of a polysaccharide such as agarose, cellulose, dextrans or sepharose;
- a material comprising or consisting of a mineral;
- a material comprising or consisting of a metal oxide such as aluminum oxide, magnesium oxide, titanium oxide or zirconium oxide;
- a material comprising or consisting of a metal such as gold or platinum; and
- a material comprising or consisting of a derivative of the foregoing.

Also, any solid support suitable for anion exchange chromatography may be used as solid support to provide a surface that allows to bind, if necessary after appropriate functionalization, inhibitors such as in particular amplification inhibitors. Exemplary polymeric materials that provide, or can be modified to provide acidic groups such as carboxyl groups include, for example, polymers such as polystyrene, latex polymers (e.g., polycarboxylate coated latex), polyacrylamide, polyethylene oxide, polyacrylate and derivatives thereof.

Preferred formats of the solid support include, but are not limited to, particles such as beads, membranes, filters, plates, columns, vessels and dipsticks. According to one embodiment, the anionic surface which binds inhibitors is provided by a vessel, for example the inner surface of a vessel that is used to receive the fixed sample and for performing the lysis. The inner surface of the surface or portions thereof can be functionalized, e.g. coated, with suitable ligands, e.g. carboxylated ligands or polyanionic polymers. Examples of respective vessels that can be respectively functionalized include, but are not limited to, microtubes and wells of a microplate. In this embodiment, the inhibitors and precipitates bind to the inner surface of the vessel and are thereby depleted from the lysate. When removing the lysate from said vessel, the bound inhibitors and precipitates remain in the vessel and are thereby separated from the lysate.

According to a preferred embodiment, the anionic surface is provided by a solid support that can be provided as suspension and can be separated from a liquid phase. Preferably, the surface which binds inhibitors is provided by particles. Preferably, particles having a carboxylated surface are used. The particles may have an average size that is selected from a range of 100 nm to 50 µm, 200 nm to 40 µm, 300 nm to 35 µm, 400 nm to 30 µm, 450 nm to 25 µm, 500 nm to 20 µm, 550 nm to 15 µm, 600 nm to 12.5 µm, 650 nm to 10 µm, 700 nm to 7.5 µm, 750 nm to 5 µm, 800 nm to 3.5 µm, 800nm to 3 µm, 800 to 2.5 µm, 800nm to 2 µm and 800 nm to 1.5 µm. Particles of the respective sizes and in particular of a smaller size such as 10µm or less, 7.5µm or less, preferably 5µm or less, 2.5µm or less or 1.5µm or less are easy to handle and can be well resuspended in the lysis mixture. Furthermore, respective small particles provide a large surface area that can bind and accordingly can efficiently deplete inhibitors and other contaminants such as precipitates from the lysate. Suitable materials for providing or making the particles and for providing a polyanionic surface are described above. The particles may also comprise more than one of the above described materials, e.g. comprising two or more layers comprising or consisting of different materials to provide the particle body.

If the particles are non-magnetic, they can be collected for example by filtration or sedimentation which can according to one embodiment be assisted by centrifugation. It is preferred though to use magnetic particles, because magnetic particles including the bound inhibitors and precipitates can be processed easily by the aid of a magnetic field, e.g. by using a permanent magnet. This embodiment is preferred as it is compatible with established systems capable of processing magnetic particles and is also suitable for automation. Here, different systems exist in the prior art that can be used in conjunction with the present invention to process magnetic particles having an anionic surface to which the inhibitors and optionally precipitates (if e.g. formed during lysis) were bound. According to one embodiment, the magnetic particles with the bound inhibitors and precipitates are collected at the bottom or the side of the reaction vessel. Thereby, bound inhibitors and precipitates are depleted from the lysate and concentrated at the bottom or side of the reaction vessel. The remaining liquid sample which corresponds to the cleared lysate can then be removed from the reaction vessel. Removal can occur e.g. by aspiration. Such systems are well known in the prior art and thus need no detailed description here. In an alternative system that is known for processing magnetic particles, a magnet which is usually covered by a cover or envelope plunges into the reaction vessel to collect the magnetic particles. The magnetic particles that carry the bound inhibitors and precipitates can then be removed, leaving behind the inhibitor depleted cleared lysate. As respective systems are well-known in the prior art and are also commercially available (e.g. QIASYMPHONY®; QIAGEN), they do not need any detailed description here. In a further alternative system that is known for processing magnetic particles, the lysate including the magnetic particles can be aspirated into a pipette tip and the magnetic particles can be collected in the pipette tip by applying a magnet e.g. to the side of the pipette tip. The remaining sample which corresponds to the inhibitor depleted, cleared lysate can then be released from the pipette tip while the collected magnet particles which carry the bound inhibitors and precipitates remain due to the magnet in the pipette tip. The collected magnetic particles can then be processed further. Such systems are also well-known in the prior art and are also commercially available (e.g. BioRobot EZ1, QIAGEN) and thus, do not need any detailed description here.

The magnetic particles can for example have superparamagnetic, paramagnetic, ferrimagnetic or ferromagnetic characteristics. Preferably, superparamagnetic particles are used. The magnetic particles may comprise a magnetic material that is incorporated in the particles and/or is associated with the particles. To avoid leaching of the magnetic material, the magnetic material is preferably completely encapsulated. The magnetic material may provide the core(s) of the particles, may be comprised in the core and/or may be applied onto the core of the particle.

According to one embodiment, the particle comprises a polymer core e.g. made of polystyrol, which is surrounded by at least one polymeric layer, preferably at least two polymeric layers, preferably a polyethylenimine layer and/or a polyacrylate layer. Preferably, the particle surface is made of or comprises a polyacrylate. Thereby, carboxyl groups are provided at the surface of the particle. Carboxyl groups are very effective in binding inhibitors, such as PCR inhibitors and are also very effective in binding precipitates that are formed during lysis. Thereby, inhibitors and precipitates can be depleted from the lysate. If magnetic particles are used, a magnetic material such as e.g. iron oxide can be deposited on the polystyrol core. Afterwards, at least one, preferably at least two polymeric layers as described above are applied and the surface of the obtained magnetic particle provides functional groups suitable for binding inhibitors, such as e.g. carboxyl groups.

For functionalizing a surface with anionic functional groups that can bind inhibitors, several methods are feasible. The functional groups, respectively compounds comprising respective groups, may be bound directly to the surface, either covalently or non-covalently, electrostatically and/or may form part of a polymer or other composition which forms a surface coating or which is provided at the surface of the solid support. Also precipitation based approaches are feasible. According to one embodiment, the functional groups such as preferably carboxyl groups are provided in form of a coating on the solid support. According to one embodiment, a covalent coupling strategy is used. Suitable methods for providing functional groups such as carboxyl groups on the surface of a solid support are well known to the skilled person and thus, do not need any specific description herein. The respective carboxyl groups are preferably provided by a polyacrylate layer or another polyanionic polymer which comprises carboxyl groups. Suitable examples were described above.

When following the teachings described herein, binding of inhibitors to the anionic surface of the solid support occurs under conditions wherein the inhibitors bind to the surface, but wherein the nucleic acids of interest do not or bind to a lesser extent. Hence, the used lysis conditions are selective in that predominantly inhibitors bind to the solid support, respectively, its anionic surface, but wherein no substantial binding of the nucleic acids of interest occurs, so that the nucleic acids remain in a sufficient amount in the lysate to allow performing the intended analytical method, such as an amplification reaction. As described above, preferably, a solid support having a carboxylated surface is used. Most preferred, magnetic particles having a carboxylated surface are used.

According to one embodiment, the solid support that is used for binding inhibitors is provided prior to, during or after step a). As discussed above, inhibitors are in particular released during heating in step b) as this heating steps effects, respectively promotes the lysis. Therefore, it is preferred to provide the solid support which preferably has an anionic surface as described above, prior to step b). Preferably, the solid support having an anionic surface is comprised in the lysis mixture. Thereby, it is ensured that inhibitors that are released upon lysis of the fixed biological sample, which in particular occurs in step b), are directly bound to the polyanionic surface of the solid support upon their release and thus are efficiently depleted from the remaining sample. This improves the depletion of the inhibitors. Clearing of the lysate as will be described in the next section may further improve the results of the analytical method, in particular the amplification reaction. If using a solid support having an anionic surface, also precipitates formed during lysis will bind to the anionic surface. Thus, as described, the lysate can not only be depleted from inhibitors such as PCR inhibitors but can also be depleted from precipitates. The solid support having an anionic surface for binding inhibitors and precipitates is preferably added prior to or in step a) and may also be comprised in the aqueous lysis composition. As described above, preferably, a solid support having a carboxylated surface is used. Most preferred, magnetic particles having a carboxylated surface are used.

### Lysate clearing

In order to improve the performance of the subsequent analytical method, in particular when performing an amplification reaction such as a PCR reaction, it is preferred to clear the lysate. According to this embodiment, at least a portion (aliquot) of the cleared lysed sample is used in the analytical method. Several options exist to clear the lysed sample. Non-limiting examples will be described below. A respective clearing step is advantageous to remove and/or inactivate precipitates that are comprised in the lysate as well as smaller tissue/cell fragments still present in the lysate after lysis procedure. However, as is shown by the presented examples, a respective clearing step is not necessarily required, in particular when using a water-soluble polyanionic polymer such as preferably polyacrylic acid.

According to one embodiment, means are provided prior to, during or after lysis for removing contaminants, in particular precipitates, thereby providing a cleared lysate. The term "removing" as used in this conjunction means that the amount of precipitates is at least reduced or otherwise inactivated to an extent that the subsequent analytical method can be adequately performed. According to one embodiment, at least a portion of the lysate is passed through means that can hold back or remove precipitates and/or other solid contaminants during passage of the lysed sample. Suitable means may be selected from the group comprising filter materials, membranes or layers or fillings of particles. The lysate may pass through said means and precipitates and/or other contaminants are caught, respectively, are held back by said means thereby providing a cleared sample once the sample has passed said means. Said means such as e.g. a filter or membrane may be porous. The pores should be sufficiently small to efficiently hold back and thus remove precipitates that are present in the lysis mixture. Said means should not substantially bind or hold back nucleic acids.

One potential source of contaminants, in particular when processing a fixed sample which comprises large amounts of cells such as a fixed tissue sample, is the formation of precipitates during lysis. Respective precipitates may in particular be formed during heating of the fixed sample in step b). The carry-over of precipitates from the lysate into the analytical method such as e.g. an amplification reaction or a detection reaction can disturb said analysis method and/or the interpretation of the obtained results and is thus not desired. Furthermore, a lysate comprising precipitates may not be appealing to the user. Therefore, it is advantageous to separate precipitates from the remaining lysate in order to provide a cleared lysate. Separation can be assisted e.g. by sedimentation or centrifugation. The precipitates may e.g. sediment at the bottom of the tube, leaving behind a supernatant which corresponds to the cleared lysate which can then be used in the analytical method such as e.g. a reverse transcription and/or amplification reaction. Sedimentation can be accelerated and improved e.g. by centrifugation. However, it is preferred to assist the separation of the precipitates and to separate at least a portion of the precipitates from the lysate prior to using the cleared lysate in a nucleic acid analysis method.

As described above, it is preferred to use a solid support which has an anionic surface for binding inhibitors either alone or, preferably, in combination with the at least one polymer which prevents or reduces the inhibition of the subsequent nucleic acid analysis method because said solid support additionally also has a lysate clearing effect. Precipitates that are formed during lysis bind efficiently to said solid support having an anionic surface. Therefore, the solid supports that are described above as suitable and preferred for depleting inhibitors by binding them to the polyanionic surface can also be used for binding precipitates and thus for lysate clearing. As described above, preferably, a solid support having a carboxylated surface is used. Most preferred, magnetic particles having a carboxylated surface are used.

According to a preferred embodiment, the fixed sample is contacted prior to, during or after lysis with at least one solid support which binds precipitates, thereby capturing the precipitates. Binding to the solid support preferably is unspecific and is achieved by adsorption. The bound precipitates are preferably separated from the remaining lysate, thereby providing a cleared lysate. Separation can be assisted by sedimentation, centrifugation or magnetic separation if a magnetic solid support is used. A magnetic solid support has magnetic properties at least in the presence of a magnetic field and thus can be moved by the aid of a magnetic field. The solid support may have superparamagnetic, paramagnetic, ferrimagnetic or ferromagnetic properties. Suitable embodiments and magnetic separation technologies were described above and it is referred to the respective disclosure. E.g. the bound precipitates can be concentrated at the bottom of a vessel and the supernatant, which corresponds to the cleared lysate, can be obtained. Binding the precipitates to the solid support assists the sedimentation and hence alleviates the removal of the precipitates from the remaining sample. However, the addition of the solid support is also beneficial and provides a clearing effect in that the inhibitory effect of comprised contaminants such as precipitates is reduced, if the bound precipitates are not separated from the lysate but hence, are present during the analytical method. Apparently, complexing the precipitates by binding them to the solid support already provides a beneficial effect, apparently because the bound precipitates are not sufficiently accessible and hence are sufficiently inactivated in that they do not severely disturb the analytical method.

According to one embodiment, the solid support is selected from the group consisting of particles, plates and other particulate matter. Other solid supports that are also suitable for binding precipitates were described above in conjunction with the solid support which provides a polyanionic surface for binding inhibitors. It is referred to the above disclosure. Respective solid supports can serve a dual function by binding inhibitors and precipitates. The term "binding" is used in a broad sense and refers to any interaction of the precipitates with the solid support that allows to separate at least a portion of said precipitates together with the solid support from the remaining sample. Binding preferably is achieved by adsorption.

Binding of precipitates to the anionic surface of the solid support occurs under conditions wherein these contaminants bind to the solid support but wherein the nucleic acids of interest do not or bind to a lesser extent. Hence, the used lysis conditions are selective in that predominantly precipitates bind, preferably adsorb, to the solid support but wherein no substantial binding of the nucleic acids of interest occurs, so that the nucleic acids remain in a sufficient amount in the lysate to allow performing the intended analytical method, such as an amplification reaction.

According to one embodiment, the solid support that is used for binding inhibitors and precipitates is provided prior to, during or after step a). As discussed above, precipitates may in particular be formed during heating in step b). Therefore, it is preferred to provide the solid support suitable for capturing precipitates that are formed during lysis prior to step b). This is particularly feasible if particles are used as solid support. The particles can be e.g. included in the lysis mixture that is provided in step a). Thereby, it is ensured that precipitates that are formed upon lysis of the fixed biological sample, which in particular occurs in step b), are directly bound to the solid support upon their formation and thus are efficiently depleted from the remaining sample. This improves the capturing of the contaminants. Clearing of the lysate may further improve the results of the analytical method, in particular the amplification reaction.

As described above, it is preferred to use a solid support having a polyanionic surface, preferably having a carboxylated surface. Respective solid supports allow to bind and thus allow to deplete inhibitors as well as precipitates. The solid support having a polyanionic surface for binding the inhibitors and precipitates may be comprised in the aqueous lysis composition. Preferably, magnetic particles having a carboxylated surface are used.

According to one embodiment, the solid support used for lysate clearing comprises or is a molecular sieve. A molecular sieve is a material containing small pores of a precise and uniform size that may be used as an adsorbent. Preferably, the molecular sieve comprises aluminosilicate minerals, clays, porous glasses, microporous charcoals, zeolites, active carbons, or synthetic compounds that have open structures through which small molecules, such as water can diffuse. Molecular sieves such as zeolites are particularly effective in removing contaminants such as precipitates from a lysate. The molecular sieve may be added to the aqueous lysis composition which preferably is a lysis solution. They may also be used in addition to the solid supports described above. When processing acidic fixed samples, using a molecular sieve such as a zeolite as solid support has the specific advantage that zeolites are capable of elevating the pH value of the lysed sample and/or the lysis mixture. This is beneficial as many analysis methods such as e.g. amplification reactions do not work properly at acidic pH values. This problem can be overcome when incorporating zeolites during the preparation of lysis mixture and in particular during preparation of a cleared lysate. E.g. the addition of zeolites allows to elevate the pH value from an acidic pH value to a pH value between 7 and 9.5, preferably 7.5 to 9, thereby additionally improving the performance of the analytical method which requires a pH value in a respective range such as e.g. an amplification reaction, in particular a PCR. Hence, according to one embodiment, at least one type of solid support is added prior, during or after lysis of the fixed sample which has the effect that the pH value of the lysed sample is elevated. Preferably, a molecular sieve, more preferred zeolites, are used for this purpose. The molecular sieve can also be added in addition to other solid supports that bind precipitates such as carboxylated particles or other carboxylated solid supports.

### Analytical methods

The lysate that is obtained with the present invention can be used directly in a nucleic acid analysis method. As described herein, "directly" in this respect means that it is not necessary to isolate and in particular to purify the nucleic acids comprised in the lysate prior to performing the subsequent nucleic acid analysis method which preferably is an amplification method. Therefore, no nucleic acid isolation or purification is performed prior to performing the nucleic acid analysis method. However, as described herein it is within the scope of the present invention to further process the lysate prior to subjecting at least an aliquot thereof to the nucleic acid analysis method. E.g. the lysate may be cleared and at least an aliquot of the cleared lysate is then used in the nucleic acid analysis method which preferably is an amplification reaction. Furthermore, the obtained lysate may also be treated with enzymes such as RNases, DNases and/or a reverse transcriptase prior to performing the nucleic acid analysis method.

According to one embodiment, an aliquot of the obtained lysate, which is optionally cleared, can be contacted with reagents that are required for performing an amplification reaction. According to one embodiment, the lysate, which is optionally cleared, is added to an aqueous solution comprising reagents that are necessary for performing an amplification reaction. The amount of used lysate, which is optionally cleared, that is added to the composition comprising the reagents for performing the analysis method such as e.g. the amplification reaction and hence is comprised in the resulting mixture can largely vary and can be chosen e.g. from 1% to 98%, 1% to 80%, 5% to 70%, 10% to 60% and 15% to 50%. Hence, the lysate which is optionally cleared, can be used in small amounts as well as in rather large amounts in the analysis reaction such as e.g. the amplification reaction. The teachings of the present invention in particular have a positive effect if a high amount of lysate is added, because the inhibition, respectively disturbance of the reaction, which results from the inhibitors comprised in the lysate, is advantageously reduced as is demonstrated by the examples.

Furthermore, a lysate that was obtained using the teachings of the present invention can also be used to reconstitute a dry composition, preferably a freeze-dried composition, comprising reagents for performing the analysis reaction such as e.g. an amplification reaction. Also in this case, at least a portion of the lysate is used in a nucleic acid analysis method in the sense of the present invention. The dry composition may comprise one or more reagents necessary for performing the intended analytical method such as an amplification reaction. Preferably, the dry composition comprises at least one polymerase. For analysing RNA, the dry composition may comprise a reverse transcriptase. According to one embodiment, the dry composition is a freeze-dried composition. Freeze-dried compositions are widely used for providing reagents necessary for amplification reactions in a storable form. Methods for preparing respective freeze-dried compositions as well as suitable additives that stabilise the comprised reaction compositions, in particular biochemical components such as proteins are well-known in the prior art (see e.g. Freeze-Drying/Lyophilization of Pharmaceutical and Biological Products, Second Edition, WO 01/92569, WO 2010/001162, US 2010/0068716 and US 2010/0159529) and thus, need no detailed description here. The dry composition that can be used in the method according to the present invention is a storable composition. Preferably, it is suitable for long-term storage. According to one embodiment, the dry composition is stable during storage for a time period of at least 3 months, at least 6 months, at least 10 months or at least 12 months. Preferably, the dry composition is stable for a time period of 3 to 18 months or 6 to 12 months. According to one embodiment, the dry composition comprises at least some of the chemical and/or biochemical reagents necessary for conducting the intended analysis method. Preferably, it comprises all of the necessary reagents because upon addition of the lysate the composition is ready for performing the analytical method. This is particularly advantageous when using the method e.g. in a LoC system. According to a preferred embodiment, the dry composition comprises at least some, preferably all, of the reagents necessary for conducting an amplification reaction, preferably a PCR reaction. As discussed above, respective dry compositions, in particular freeze-dried compositions, are widely used to provide the reagents necessary for an amplification reaction in form of a so-called master mix, in a storable form. When intending to perform the amplification reaction, the dry composition only needs to be reconstituted using the lysate to form the amplification reaction mixture, wherein, however, optionally further reagents can be added e.g. if not all reagents were already comprised in the dry composition, what is, however, preferred. Preferably, the dry composition comprises one or more, preferably all reagents selected from the group consisting of a polymerase, a reaction buffer suitable for performing an amplification reaction and dNTPs. Preferably, it also comprises primers and/or labelled probes which allow e.g. the detection the presence or absence of one or more target nucleic acids that are, e.g., indicative of a certain disease or infection. However, primers and/or probes may also be added separately. After reconstitution using the lysate and optionally the addition of further additives, such as primers and/or probes, the resulting reconstituted composition is ready for performing the amplification of a target nucleic acid. According to one embodiment, at least 60%, at least 70%, at least 80%, at least 90%, at least 95% or 100% of the liquid that is used for reconstitution is provided by the lysate. Reconstitution can be advantageously achieved exclusively by addition of the lysate. According to one embodiment, at least an aliquot of the lysate, which optionally is further processed such as cleared in advance, that was obtained as described above, is contacted with the dry composition for reconstitution. In order to ensure that the reconstituted composition comprises the reagents in a concentration appropriate for the intended analysis method, a predetermined amount of lysate is added to the dry composition. Said predetermined amount is chosen such so that in the reconstituted composition, the reagents are comprised therein in a concentration suitable for performing the amplification reaction. The reconstitution process can be assisted by agitation e.g. by pipetting the resulting mixture up and down, stirring, shaking or vortexing.

The analytical method may be any chemical and/or biotechnological method that can be used to analyse nucleic acids e.g. in order to amplify, identify, detect and/or quantify a nucleic acid. Preferably, the analytical method comprises a detection reaction which allows to detect the presence, absence and/or quantity of nucleic acids comprised in the lysate. Preferably, said method comprises the amplification of at least one target nucleic acid and the subsequent detection of the generated amplicon using e.g. labelled probes. Respective analytical methods are well-known in the prior art and are also commonly applied in the medical, diagnostic and/or prognostic field in order to analyse nucleic acids or a specific nucleic acid comprised in a sample. Hence the analytical method may comprise an analysis of nucleic acids comprised in the lysate to identify the presence, absence and/or severity of a disease state including but not being limited to a multitude of neoplastic diseases, in particular premalignancies and malignancies such as different forms of cancers. E.g. the lysate can be analysed in order to detect diagnostic and/or prognostic markers (e.g., tumor-derived nucleic acids) in many fields of application, including but not limited to disease screening, oncology, cancer screening, early stage cancer screening, premalignancy screening, cancer therapy monitoring, genetic testing (genotyping), infectious disease testing, testing for pathogens, injury diagnostics, trauma diagnostics, transplantation medicine or many other diseases and, hence, are of diagnostic and/or prognostic relevance.

Basically, any nucleic acid analysis method can be performed on the obtained lysate including, but not limited to, identification technologies, amplification technologies, polymerase chain reaction (PCR), isothermal amplification, reverse transcription polymerase chain reaction (RT-PCR), real time PCR, quantitative real time polymerase chain reaction (qPCR), fluorescence detection, hybridization assays, DNA or RNA sequencing, next generation sequencing, restriction analysis, reverse transcription, enzymatic digestions, such as DNase or RNase digestions, NASBA, LAMP (loop mediated isothermal amplification), RPA (recombinase polymerase amplification), tHDA (helicase dependent amplification), NEAR (nicking enzyme amplification reaction), TMA (transcription mediated amplification) and NASBA (nucleic acid sequence based amplification), allele specific polymerase chain reaction, polymerase cycling assembly (PCA), asymmetric polymerase chain reaction, linear after the exponential polymerase chain reaction (LATE-PCR), , hot-start polymerase chain reaction, intersequence-specific polymerase chain reaction (ISSR), inverse polymerase chain reaction, ligation mediated polymerase chain reaction, methylation specific polymerase chain reaction (MSP), multiplex polymerase chain reaction, nested polymerase chain reaction, solid support polymerase chain reaction, detection technologies using labeled, preferably fluorescently labeled primers and/or probes, or any combination of the foregoing. Respective technologies are well-known to the skilled person and thus, do not need further description here. Preferably, the analytical method is or comprises a nucleic acid amplification method.

### Nucleic acids

The term "nucleic acid" or "nucleic acids" as used herein, in particular refers to a polymer comprising ribonucleosides and/or deoxyribonucleosides that are covalently bonded, typically by phosphodiester linkages between subunits, but in some cases by phosphorothioates, methylphosphonates, and the like. Nucleic acids include, but are not limited to all types of DNA and/or RNA, e.g. gDNA; circular DNA; plasmid DNA; circulating DNA; PNA; LNA, cyclohexene nucleic acids; RNA/DNA hybrids; hnRNA; mRNA; noncoding RNA (ncRNA), including but not limited to rRNA, tRNA, miRNA (micro RNA), siRNA (small interfering RNA), snoRNA (small nucleolar RNA), snRNA (small nuclear RNA), pwi-interacting RNA (piRNA), repeat associated RNA (rasiRNA), as RNA and stRNA (small temporal RNA); fragmented nucleic acids; nucleic acid obtained from subcellular organelles such as mitochondria or chloroplasts; and nucleic acid obtained from pathogens, microorganisms, parasites, or DNA or RNA viruses that may be present in a biological sample, e.g. bacteria, viral or fungi nucleic acids; synthetic nucleic acids, extracellular nucleic acids.

### Samples and fixation

The term "biological sample" is used herein in a broad sense and is intended to include sources that contain nucleic acids and can be fixed. Exemplary biological samples include, but are not limited to tissues, including but not limited to, liver, spleen, kidney, lung, intestine, thymus, colon, tonsil, testis, skin, brain, heart, muscle and pancreas tissue, cell containing samples that can be fixed, body fluids and samples derived therefrom, aspirates, cell culture, bacteria, microorganisms, viruses, plants, fungi, biopsies, bone marrow samples, swab samples, organ samples, faeces, skin fragments and organisms. Materials obtained from clinical or forensic settings or environmental samples that contain or are suspected to contain nucleic acids are also within the intended meaning of the term biological sample. Preferably, the sample is derived from a human or animal. Preferably, the biological sample is a tissue sample, preferably an organ tissue sample, most preferred obtained from a human or rodent. The tissue may be obtained from autopsy, biopsy or from surgery. It may be a solid tissue such as, for example, parenchyme, connective or fatty tissue, heart or skeletal muscle, smooth muscle, skin, brain, kidney, liver, spleen, breast, carcinoma (e.g. bowel, nasopharynx, breast, lung, stomach etc.), cartilage, lymphoma, meningioma, placenta, prostate, thymus, tonsil, umbilical cord or uterus. The tissue may be a tumor (benign or malignant), cancerous or precancerous, obtained from an animal or human subject affected by disease or suspected of same (normal or diseased), or be affected by other pathology. It may be obtained by autopsy, biopsy (e. g., endoscopy or laparoscopy), or surgical resection.

Fixation of the biological sample can be effected with any fixative known to the person skilled in the art, in particular with acids, alcohols, ketones or other organic substances, such as, in particular, glutaraldehyde, formaldehyde or paraformaldehyde. Examples of fixatives and uses thereof may be found in Sambrook et al. (2000); Maniatis et al. (1989). Preferably, the used fixation also preserves DNA and RNA. According to one embodiment, the used fixation stabilizes the morphology of the sample also at ambient temperatures at least for several weeks or months. According to one embodiment, the fixative has a crosslinking effect such as formaldehyde and paraformaldehyde. Biological samples fixed with formaldehyde can be efficiently processed and thus lysed with the method according to the present invention. According to one embodiment of the process according to the invention, a formaldehyde-fixed, paraffin-embedded biological sample (FFPE sample) is used. Other fixatives and fixation methods for providing a fixed biological sample are known in the prior art. In particular alcohol based fixed samples can be used. A respective fixation process involving alcohols is e.g. described in WO 2008/104564, herein incorporated by reference. As is shown by the examples, samples fixed according to said procedure can be efficiently lysed with the method according to the present invention and the obtained lysate can be used directly in downstream analytical methods such as amplification reactions.

Furthermore, the fixed tissue may or may not be embedded in a non-reactive substance such as paraffin. Embedding materials include, but are not limited to, paraffin, mineral oil, non-water soluble waxes, celloidin, polyethylene glycols, polyvinyl alcohol, agar, gelatine, nitrocelluloses, methacrylate resins, epoxy resins or other plastic media. Thereby, one can easily produce tissue sections of the biological material suitable for histological examinations. Said embedding steps preferably are carried out under mild conditions, e.g. with low melting paraffin (wax). Said steps can be carried out manually or by any automated system. Methods and compositions of the invention can be applied to any fixed cell or tissue sample, whether it has been embedded or not.

According to one embodiment, the biological sample is fixed with formalin, in particular after the sample has been embedded in a non-reactive substance such as paraffin. Tissue sections can then be prepared from the paraffin embedded sample by means of suitable cutting devices, for example by means of a microtome, the thickness of these sections normally being about 5 to 20 µm for examination under a light microscope. Furthermore the paraffin-embedded sample can also be reduced in size by other methods, for instance by perforating with a hollow needle or by the so-called laser capture method, to give smaller sample fragments.

When processing fixed samples that have been embedded in an embedding material such as paraffin, it is within the scope of the present to include a step to remove said embedding material prior to step a). Thus, according to one embodiment wherein the fixed biological sample is a biological sample embedded in an embedding material such as paraffin, the embedding material is at least partly, preferably completely, removed from the fixed biological sample before contacting the sample with the aqueous lysis composition in step a). The removal of the embedding material such as paraffin from the biological sample can in principle take place by all methods known to the skilled worker for deparaffinization of biological samples. The deparaffinization preferably takes place by initially contacting the sample with a hydrophobic organic solvent, in particular with an aromatic hydrocarbon, such as xylene, in order to dissolve out the embedding material such as paraffin. Suitable deparaffinization methods are e.g. described in WO 2011/104027, WO 2011/157683 and WO 2007/068764. After removal of the embedding material the sample it may be rehydrated, this rehydration may e.g. take place by stepwise washing with aqueous alcohol solutions with decreasing alcohol concentration (=descending alcohol series).

However, as is shown by the examples, it is not required to remove the non-reactive embedding material such as paraffin prior to step a), if a respectively embedded fixed sample is processed using the method according to the present invention. E.g. during step b), the sample is automatically deparaffinized as the paraffin melts. The paraffin forms a layer or small chunks on the lysate and resolidifies upon cooling of the lysate. The lysate - which is free of paraffin - can then be obtained e.g. by pipetting through said paraffin layer. It is a great advantage that it is not necessary to include a separate embedding material removal step in the method according to the present invention as this saves time and chemistry and contributes to that the method can be performed very rapidly and cost-efficient and furthermore, is suitable for automation. The same applies to other embedding materials besides paraffin which melt under the conditions used in step b).

The method according to the present invention does not require a step wherein the nucleic acids are isolated from the lysed sample e.g. by binding them to a solid support and/or by precipitating them out of the lysate in order to purify them prior to using them in an analytical method. Rather, inhibitors that could interfere with the intended analysis method and preferably also precipitates are depleted as described above using the polymer and/or the solid support. This ensures that the analysis method can be efficiently performed even if a large amount of the lysate, which preferably is a cleared lysate, is used. Hence, e.g. the analysis method may be carried out in immediate succession after lysis using an aliquot of the lysate, which optionally but preferably is cleared, without the need for performing a purification of the nucleic acids between lysis and analytical method. Therefore, the method is also particularly suitable for use in processing cartridges e.g. in LoC (Lab on Chip) approaches.

Furthermore, the method according to the present invention does in contrast to prior art methods not require the use of a proteolytic enzyme such as e.g. subtilisins, proteinase K, trypsin and other enzymes. This is again a great advantage, because the use of respective enzymes is costly and furthermore, often prolongs the overall preparation time as the enzymatic digestion step requires longer incubation times at moderately elevated temperatures. The present invention, wherein no proteolytic enzyme is used for lysing the sample, therefore has important advantages.

Furthermore, the present invention has the advantage that no chaotropic agents such as guanidine salts, thiocyanate salts, isothiocyanate salts, perchlorates, iodide salts and urea are required for lysing the sample and thus are preferably also not used. In fact, the method according to the present invention does not require the use of any chaotropic salts or chaotropic agents. This is a further important advantage, as the method according to the present invention can be carried out without the use of respective toxic agents.

As described above, the resulting lysate can be used directly in a method for analysing nucleic acids, such as in particular an amplification reaction. Contaminants that are comprised in the lysate due to the lysis of the sample such as in particular proteoglycans, proteins, metabolites and sugars are advantageously depleted. Thereby, a lysate is provided that can be used directly in a nucleic acid analysis method, such as an amplification based method, thereby overcoming the need to isolate and purify the nucleic acids first. Hence, "directly" in this respect means that it is not necessary to isolate and in particular to purify the nucleic acids comprised in the lysis mixture prior to performing the subsequent nucleic acid analysis method which preferably is an amplification method. Therefore, no nucleic acid isolation or purification is performed prior to performing the nucleic acid analysis method. However, as described below it is within the scope of the present invention to further process the lysate prior to subjecting the lysate or an aliquot thereof to the nucleic acid analysis method. E.g. the lysate may be cleared and the cleared lysate or an aliquot thereof is then used in the nucleic acid analysis method which preferably is an amplification reaction. Due to the efficient depletion of inhibitors comprised in the lysate, also increased amounts of lysate can be used in the subsequent nucleic acid analysis method. This is an important advantage as the sensitivity is increased because larger amounts of nucleic acids are subjected to the analysis method. This higher amount of nucleic acids may also compensate for a potential inhibition of the analytical reaction such as an amplification reaction by contaminants that may be still comprised in the lysate.

According to a second aspect, a nucleic acid analysis method is provided, comprising
a) lysing a sample comprising nucleic acids according to the method of the first aspect of the present invention,
b) using at least a portion of the lysate in a nucleic acid analysis method.

Details with respect to step a) were described above in conjunction with the method according to the first aspect of the present invention. It is referred to the above disclosure which also applies here. Preferably, the lysate is cleared as described above and a portion of the cleared lysed sample is used in step b). Preferably, a solid support having an anionic surface, preferably having a carboxylated surface, is used for binding inhibitors as well as precipitates, thereby also clearing the lysate. The solid support is preferably used in addition to the polymer which prevents or reduces the inhibition of the nucleic acid analysis method, which preferably involves an amplification step. Said solid support is preferably separated prior to step b).

Suitable nucleic acid analysis methods that can be performed in step b) were also described above. It is referred to the respective disclosure which also applies here. Preferably, an amplification reaction is performed. If the nucleic acid of interest is RNA, it is preferred to first perform a reverse transcription step to generate cDNA prior to performing the amplification. The lysate that is obtained with the present invention allows a reverse transcription directly on the obtained lysate. There is no necessity to isolate the RNA in advance. Furthermore, a DNase digest (if the target nucleic acid is RNA) or an RNase digest (if the target nucleic acid is DNA) may be performed prior to amplification. The obtained lysate is also suitable for these purposes. Respectively processed lysates are also encompassed by the term lysate as used herein.

In a respective amplification step that may be performed in step b), nucleic acids or specific target nucleic acids comprised in the lysate are multiplied by means of a method which amplifies the nucleic acids. Such methods are well-known in expert circles and are not limiting for the invention. A preferred example for such a method is a polymerase chain reaction (PCR), if appropriate with a preceding reverse transcription in case the nucleic acid of interest is RNA. The amplification may be performed with specific primers or with unspecific primers. By using specific primers, it is possible to enhance either one or more specific gene sequence(s) from the genetic material, or, when using specific primers according to international standard, a genetic fingerprint may be generated. In addition, it is also possible to use unspecific primers, e.g. when comparing a known sample with an unknown sample according to the size pattern of the unspecific amplicons in order to find out about the origin of the unknown sample.

As discussed above, it is not necessary to isolate and in particular to purify the nucleic acids comprised in the lysate prior to performing the nucleic acid analysis method. Therefore, no nucleic acid isolation method is performed between steps a) and b). However, as described above, it is within the scope of the present invention to further process the lysate prior to subjecting at least an aliquot thereof into the nucleic acid analysis method. E.g. the lysate may be cleared and an aliquot of the cleared lysate is then used in the nucleic acid analysis method. The clearing may involve e.g. the removal of precipitates. This can be achieved e.g. by sedimenting the precipitates what may be assisted by centrifugation. Alternatively, precipitates may be removed e.g. by using a filter or by adding a solid support that binds inhibitors, in partiuclar PCR inhibitors, and precipitates. Preferably, lysate clearing is performed using a solid support having an anionic surface, as such surface also depletes inhibitors that are released during lysis of the sample. Details were described above, it is referred to the respective disclosure. According to one embodiment, however, the lysate is not cleared prior to step b). Further intermediate steps that may or may not be performed between steps a) and b) include enzymatic processes such as reverse transcription, DNase digests, RNase digests and the like.

Numeric ranges described herein are inclusive of the numbers defining the range. The headings provided herein are not limitations of the various aspects or embodiments of this invention which can be read by reference to the specification as a whole. The term "solution" as used herein, in particular refers to a liquid composition, preferably an aqueous composition. It may be a homogenous mixture of only one phase but it is also within the scope of the present invention that a solution that is used according to the present invention comprises solid components such as e.g. precipitates. The term "carboxylate" as used herein also refers to protonated carboxylate groups, e.g. carboxyl groups, since depending on the pH value of the lysis mixture always a certain amount, preferably a minor amount, of the carboxylate groups is protonated. Thus, as used herein, the terms "carboxylate" "carboxyl group" are used interchangeably and shall refer to the carboxyl group (COOH), the carboxylate anion (COO⁻) or the carboxylate salt [-COO⁽⁻⁾ X⁽⁺⁾]. Likewise, the term "acid" as used herein also refers to the salts of the respective acid, i.e. to the deprotonated form of the acid and vice versa. Thus, contacting the sample with at least one water-soluble polyanionic polymer e.g. also comprises adding a respective polymer with anionizable groups which becomes a polyanionic polymer under the conditions used during lysis and thus e.g. in the lysis mixture.

According to one embodiment, subject matter described herein as comprising certain steps in the case of methods or as comprising certain ingredients in the case of compositions, solutions and/or buffers refers to subject matter consisting of the respective steps or ingredients. It is preferred to select and combine preferred embodiments described herein and the specific subject-matter arising from a respective combination of preferred embodiments also belongs to the present disclosure.

This invention is not limited by the exemplary methods and materials disclosed herein. The invention will now be described by the following, non-limiting examples.

### EXAMPLES

### Example 1: Optimization of the pH value in the aqueous lysis composition

In order to be able to use the obtained lysate directly in a quantitative PCR reaction, it is necessary that the pH value of the obtained lysate either corresponds to the pH value required for the polymerase used in the PCR reaction or that the lysate does not negatively influence the pH value of the used PCR buffer. To directly obtain a lysate which has an appropriate pH value for this purpose is advantageous, because it makes pH adjustment after lysis obsolete. Therefore, different pH values were tested in the aqueous lysis composition in order to analyze the effect on the subsequent quantitative PCR. The aqueous lysis composition comprised as polymer for reducing PCR inhibitors PVP and two non-ionic detergents. The tested lysis compositions are shown in table 1.

**Table 1: Aqueous lysis compositions having different pH values**

| | **1** | **2** | **3** | **4** | **5** | **6** |
|---|---|---|---|---|---|---|
| PVP MW 10.000 | 0.1% | 0.1% | 0.1% | 0.1% | 0.1% | 0.1% |
| Tween-20 | 0.45% | 0.45% | 0.45% | 0.45% | 0.45% | 0.45% |
| NP40 | 0.45% | 0.45% | 0.45% | 0.45% | 0.45% | 0.45% |
| **40 mM Tris/Cl** | **pH 8.0** | **pH 8,2** | **pH 8,4** | **pH 8,6** | **pH 8,8** | **pH 9** |
| 1 mM EDTA | 1mM | 1mM | 1mM | 1mM | 1mM | 1mM |

The sample material was 6 x 10 µm tissue sections of FFPE fixed rat liver. The respectively fixed biological sample was processed as follows. One tissue section was contacted with 250 µl lysis buffer (see table 1) and 625 µg pre-separated magnetic SeraMag beads (magnetic polymer beads comprising carboxyl groups at their surface). The respectively obtained lysis mixture was incubated for 30 minutes at 95°C in a water bath to lyse the sample. The obtained lysate was vigorously mixed for 2 seconds and then cooled for one to five minutes at room temperature until the paraffin resolidifies. Afterwards, a magnetic separation step (one minute) was performed in order to remove the magnetic SeraMag beads to which PCR inhibitors and precipitates comprised in the lysate were bound. Thereby, the lysate was cleared. An aliquot of the supernatant (without the paraffin layer) was transferred into a new reaction vessel. 5 µl of the respectively obtained cleared lysate was used per 25 µl 18S qPCR Rxn.

**qPCR Rxn:**

| | **Volumen [µl]** |
|---|---|
| QuantiFast Probe MM | 12.5 |
| 18S Fwd Primer (10µM) | 1 |
| 18S Rev Primer (10µM) | 1 |
| 20x EvaGreen | 1.25 |
| Lysate | 5 |
| Water | 4.25 |

Cycling: 5 minutes 95°C, 40 x {10s 95°C, 30s 55°C, 30s 72°C}

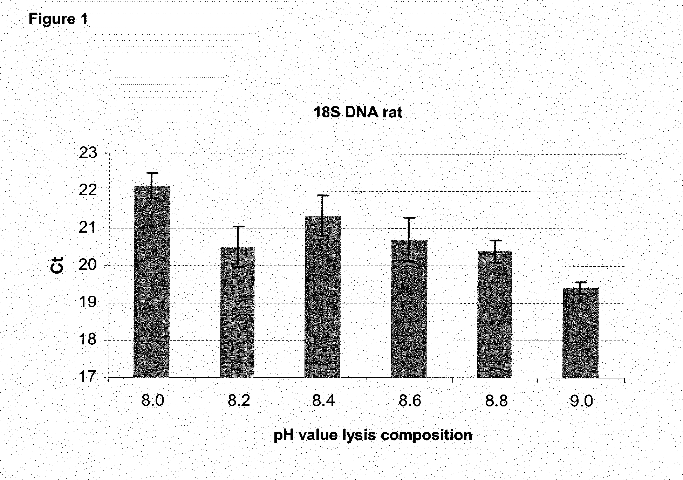

Figure 1 shows the obtained Ct values of the qPCR reaction as mean value of duplicates. A dependency of the obtained Ct values on the pH value of the lysis composition was observed. The lysis composition having the highest pH value (pH 9.0) provided the lowest Ct values and thus the best results and furthermore, achieved the lowest deviation in the tested duplicates. Therefore, a pH value of 9 is particularly suitable to prepare a lysate which can be used directly in a PCR amplification without the need to adjust the pH value of the lysate in advance. This buffer was also used in the following experiments, if not stated otherwise.

### Example 2: Validation of the heat incubation step

In this example, 8 x 10 µm tissue sections of FFPE fixed rat liver were used. The samples were processed as follows. One tissue section was contacted with 250µl lysis buffer and 625 µg of a pre-separated SeraMag beads. Four lysis mixtures were prepared. Two of the respectively obtained lysis mixtures were incubated at 95°C in a water bath either for 15 minutes or for 30 minutes. The obtained lysates were vigorously mixed for 2 seconds and then cooled at room temperature for one minute. Afterwards, a magnetic separation step (one minute) was performed in order to remove precipitates and other contaminants that were bound to the magnetic particles, thereby clearing the lysate. The respectively obtained cleared lysate (without the paraffin layer) was transferred into a new reaction vessel. Five µl of the respectively obtained cleared lysate was used per 25 µl 18s qPCR reaction.

For comparison, four tissue sections were prepared according to the QIAamp ® DNA FFPE Tissue Handbook. The handbook was followed except that for deparaffinization, the Deparaffinization Solution (QIAGEN) was used instead of xylol (one tissue section was contacted with 160µl Deparaffinization Solution, incubation for three minutes at 56°C, addition of 180 µl buffer ATL and 20µl proteinase K, the further steps (11 et seq) were then performed as described in the handbook). The nucleic acids were eluted in 50µl water. 1µl of the respectively obtained eluate was used in a 25µl 18S qPCR reaction.

**qPCR reaction:**

| | **Direct Lysis** | **QIAamp** |
|---|---|---|
| | **Volume [µl]** | **Volume [µl]** |
| QuantiFast Probe MM | 12.5 | 12.5 |
| 18S Fwd Primer (10µM) | 1 | 1 |
| 18S Rev Primer (10µM) | 1 | 1 |
| 20x EvaGreen | 1.25 | 1.25 |
| Lysate or Eluate | 5 | 1 |
| Water | 4.25 | 8.25 |

Cycling: 5 minutes at 95°C, 40 x {10s 95°C, 30s 55°C, 30s 72°C}.

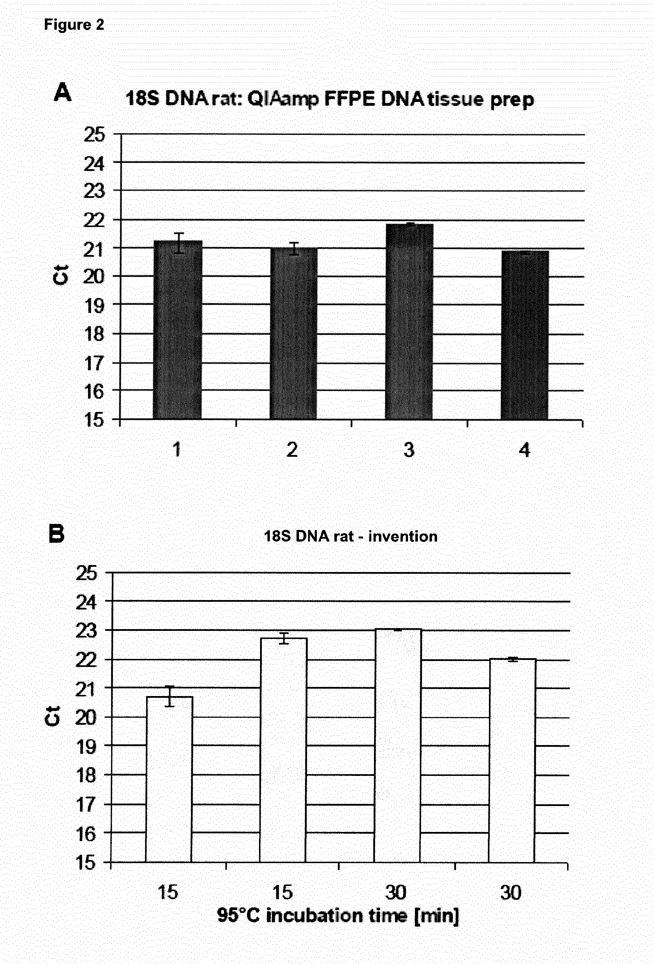

Figure 2 shows the obtained Ct values of the qPCR reaction as mean value of the duplicates for (A) nucleic acids extracted using the QIAamp FFPE Tissue Kit and (B) the lysate that was obtained with the method of the present invention at either 15 minutes or 30 minutes incubation time at 95°C. When comparing the Ct values obtained with the QIAamp isolated DNA and the results obtained with the present invention, it can be seen that with the present invention, there were more differences in the Ct values than with the QIAamp isolated DNA. However, comparing the incubation times at 95°C of the method of the present invention, there was no significant difference on average between 15 minutes and 30 minutes incubation time. Therefore, it was found that the method of the present invention provides an approximately similar result to the one that was obtained with the more time-consuming QIAamp FFPE Tissue Kit. The significant advantage of the present invention is the remarkable reduced preparation time. While the QIAamp FFPE DNA Tissue Kit requires approximately two hours and thirty minutes for preparing the DNA for PCR, the method of the present invention only takes approx. 20 to 40 minutes, depending on the used incubation time. This remarkable time-saving and the obtained comparable, stable amplification results emphasize the advantages of the present invention. The method according to the present invention is particularly suitable for high-throughput applications. However, when comparing the obtained results, it must be kept in mind that the DNA preparations (QIAamp) and the lysates (invention) that were used in the PCR reactions were obtained from single tissue sections which may comprise diverging amounts of tissue and thus nucleic acids per section. Therefore, as potentially not an identical amount of input material was used, the obtained results merely allow an approximate comparison.

### Example 3: Analysis of the effect of paraffin removal

As sample material, 6 x 10 µm tissue sections of FFPE fixed rat liver were used. The following protocols were used. One tissue section was contacted with 250µl lysis buffer and 625 µg pre-separated SeraMag beads. Six lysis mixtures were respectively prepared. Three of the respective lysis mixtures were contacted with 160 µl Deparaffinization Solution (QIAGEN) and were incubated for 5 minutes, 10 minutes or 15 minutes at 95°C for deparaffinization and lysis. The remaining three lysis mixtures were incubated directly for 5 minutes, 10 minutes or 15 minutes at 95°C. The obtained lysates were cooled down at room temperature for one minute. Afterwards, a magnetic separation step was performed (1 minute) in order to clear the lysate from precipitates and other contaminants present in the lysate. The aqueous supernatant (without the paraffin layer) corresponding to the cleared lysate was transferred into a new reaction vessel. Subsequently, 5 µl lysate were used in a 25 µl ß-actin qPCR reaction.

**qPCR reaction:**

| | **Volume [µl]** |
|---|---|
| QuantiFast Probe MM | 12.5 |
| beta-Actin Fwd Primer (10µM) | 1 |
| beta-Actin Rev Primer (10µM) | 1 |
| 20x EvaGreen | 1.25 |
| Lysate | 5 |
| Water | 4.25 |

Cycling: 5 minutes 95°C, 40 x {10s 95°C, 30s 55°C, 30s 72°C}.

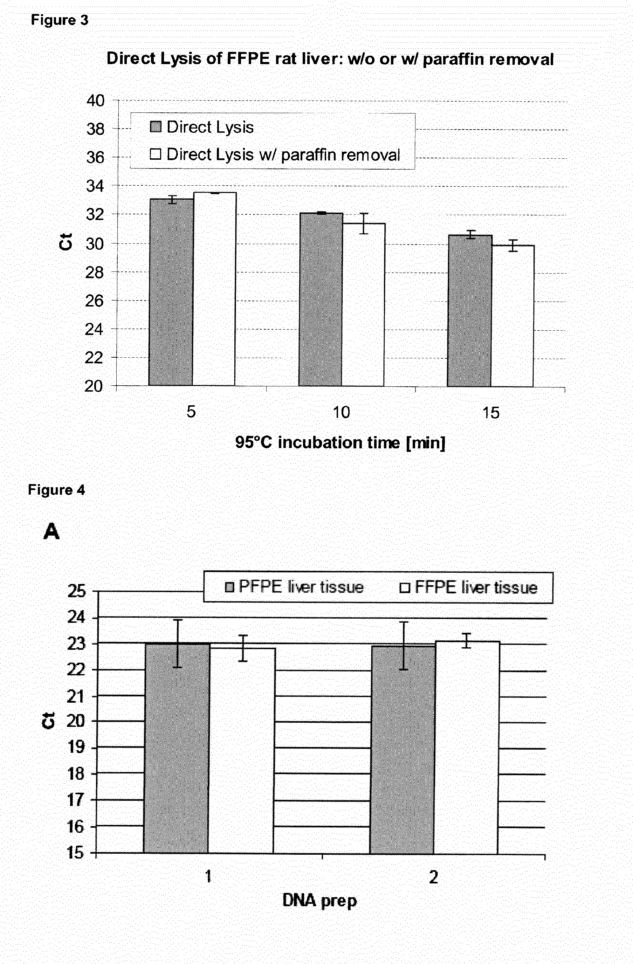

Figure 3 shows the obtained Ct values of the qPCR reaction as mean value of the prepared duplicates with the method of the present invention either with or without prior removal of paraffin. As can be seen, independent of the method used, a dependency on the incubation time was seen. A reduction in the incubation time during heating step b) goes along with an increase in the obtained Ct values. Thus, less nucleic acids are released and the detection is less sensitive. However, no beneficial effect can be seen if the paraffin is removed prior to lysis. Therefore, in order to save chemicals and hands-on time, it is preferred to not include a separate paraffin removal step.

### Example 4: Validation of the method for PFPE tissue

Sample material: 2 x 10µm tissue sections of FFPE fixed rat liver; 2 x 10µm tissue sections rat liver PFPE (Paxgene-fixed, paraffin-embedded; fixation described in WO 2008/104564); 4 x 10µm tissue section PFPE fixed rat kidney.

The following protocol was used. One tissue section was contacted with 250µl lysis buffer and 625 µg pre-separated SeraMag beads. Overall, eight lysis mixtures were prepared. 2 x FFPE rat liver, 2 x PFPE rat liver and 2 x PFPE rat kidney tissue was incubated for 15 minutes and 2 x PFPE rat kidney was incubated 30 minutes, all at 95°C, in a water bath. The obtained lysate was cooled at room temperature for one minute and the magnetic particles were magnetically separated (1 minute) in order to remove precipitates and/or contaminants. The supernatant (without the paraffin layer) which corresponds to the cleared lysate was transferred into a new reaction vessel. 5 µl of the respectively obtained lysate was used per 25µl β-actin qPCR reaction using the above-described conditions.

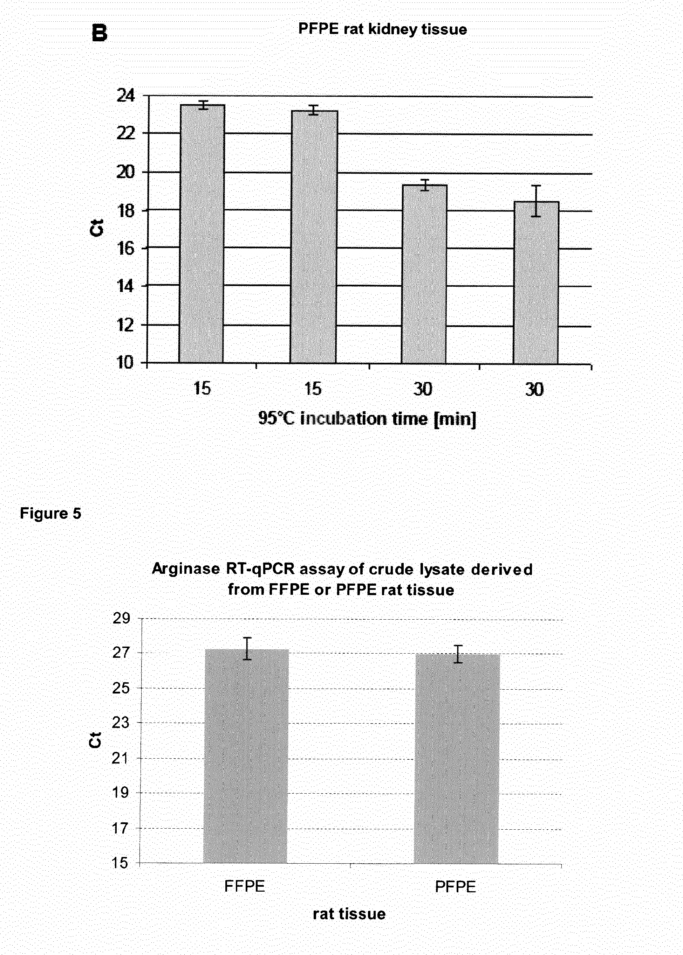

Figure 4 shows the obtained Ct values of the qPCR reaction as mean value of the duplicates. Figure 4A compares the method according to the present invention for PFPE or FFPE rat liver tissue as fixed sample material. Figure 4B shows the influence on the incubation duration on the lysis efficiency when using PFPE rat kidney tissue as sample material.

As can be seen, the method according to the present invention works equally well for FFPE as for PFPE tissue samples. Furthermore, it was found based upon the obtained Ct values for the PFPE rat kidney samples that a longer incubation duration of 30 minutes at 95°C compared to the incubation duration of 15 minutes resulted in a significant improvement of the lysis efficiency (see Figure 4B). Therefore, it is assumed that depending on the tissue type, it is recommendable to test for the optimal incubation duration for lysis at 95°C in order to obtain an optimized lysis efficiency and thus release of the nucleic acids.

### Example 5: Validation of the method of the present invention for PFPE or FFPE tissue as template for reverse transcription PCR

The aim of this example was the amplification of RNA. Here, it must be considered that the starting material comprises RNA as well as DNA and, accordingly, that RNA and DNA is comprised in the lysate. Thus, the assay must be chosen such that detection of DNA is excluded in the RT-qPCR, in order to prevent obtaining false positive results. Thus, in this example the primers were chosen such that an amplification of DNA was prevented.

As sample material, 2 x 10µm tissue sections of FFPE fixed rat liver and 2 x 10µm tissue sections of PFPE fixed rat liver were used. One tissue section was contacted with 250µl lysis buffer and 625 µg pre-separated SeraMag beads. Overall, four lysis mixtures were prepared and incubated for 15 minutes at 95°C. The lysates were allowed to cool at room temperature for one minute. Afterwards, a magnetic separation step was performed (one minute) in order to remove the magnetic beads to which the precipitates another contaminates comprised in the lysates were bound. The respectively obtained cleared supernatant (without the paraffin layer) was transferred into a new reaction vessel. 5µl of the cleared lysate were used per 25µl arginase qPCR reaction.

**qPCR reaction:**

| | **Volume [µl]** |
|---|---|
| QuantiTect Virus Kit | 5 |
| RTase | 0.25 |
| Arginase Fwd Primer (10µM) | 1 |
| Arginase Rev Primer (10µM) | 1 |
| 20x EvaGreen | 1.25 |
| Lysate | 5 |
| Water | 11.5 |

Cycling: 10 minutes 50°C, 5 minutes 95°C, 40 x {10s 95°C, 30s 58°C}.

Figure 5 shows the obtained Ct values of the RT (reverse transcription)-qPCR reaction as mean value of the tested duplicates. As can be seen, in the RT-qPCR reactions, similar results were obtained starting from the FFPE or PFPE lysate. Thus, this example shows that with the lysate that is obtained with the method of the present invention can be directly used in a RT-qPCR. Thus, RNA is released and is accessible for RNA analysis, here by performing a RT-PCR. Starting from FFPE and PFPE tissue comparable results were achieved.

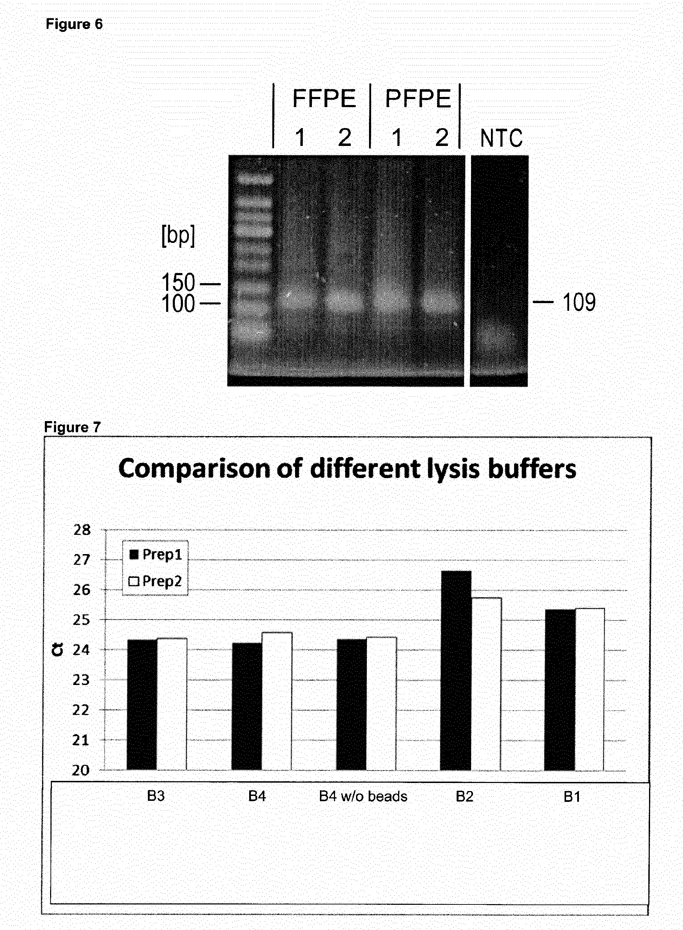

In order to test the specificity of the arginase RT-qPCR, 4µl of the first of the two RT-qPCR reactions were applied onto an agarose gel (2%). Compared to the control NTC (no template control), the specific amplicon with a 109bp fragment length was detected. The respective results are shown in Figure 6.

Figure 6 shows an agarose gel wherein the PCR products of the RT-qPCR reactions of the arginase assay was applied.

### Example 6: Influence of the used polymer on the depletion efficiency

Four different aqueous lysis compositions were tested:

| **Buffer 1 (B1)** | **Buffer 2 (B2)** | **Buffer 3 (B3)** | **Buffer 4 (B4)** |
|---|---|---|---|
| 0.1 % PVP (MW 10.000) | | 0.1 % PVP (MW 10.000) | 0.04 % PAA (MW 250k) |
| 0.45 % Tween-20 | 0.45 % Tween-20 | 0.45 % Tween-20 | 0.45 % Tween-20 |
| 0.45 % NP40 | 0.45 % NP40 | 0.45 % NP40 | 0.45 % NP40 |
| 40 mM Tris/Cl, pH 9 | 40 mM Tris/Cl, pH 9 | 40 mM borate, pH 9 | 40 mM Tris/Cl, pH 9 |
| 1 mM EDTA | 1 mM EDTA | 1 mM EDTA | 1 mM EDTA |

In separate reaction tubes comprising each 625 µg of Sera Mag Beads, 10 µm sections from rat liver tissue (PFPE - see above) were lysed in 250 µl of buffers 1 to 4. In addition, tissue samples were also lysed in the presence of buffer 4 but without the addition of the carboxylated beads. All reactions were performed in duplicates. The lysis mixtures were incubated for 15 min at 95°C using a thermomixer, followed by about 5 minutes cooling to room temperature. Beads were separated using magnetic force for about 1 minute, and the supernatant which corresponds to the cleared lysate was transferred to a new tube. The lysate was analyzed by PCR using ß-actin as target.

For PCR detection, the following reaction mixture was used: 12.5 µl QF Probe MM; 1 µl fwd primer (10 µM); 1 µl rev primer (10 µM); 1.25 µl probe (10 µM); 5 µl lysate; 4.25 µl water. PCR was performed using 95°C for 5 min (initial denaturation), followed by 40 cycles of denaturation at 95°C for 10 seconds, annealing at 55°C for 30 seconds and extension at 72°C for 30 seconds. The results are shown in Fig. 7. It was found that the lysis buffer 3 (B3) comprising borate as buffering agent yielded a better PCR performance than lysis buffer 1 (B1) comprising Tris, as can be seen by the lower Ct value. Comparing the results obtained for lysis buffer 4 (B4, comprising polyacrylic acid (PAA) and thus a water-soluble polyanionic polymer comprising carboxylate-containing monomers) and lysis buffer 1 (B1, comprising PVP), lysis performance of lysis buffer 4 (B4) was also very good. In addition, inhibitors were considerably more efficiently depleted when using lysis buffer 4 (B4) as compared to lysis buffer 1 (B1). This shows that PAA is more effective in depleting inhibitors than PVP. Better results were obtained with PAA even if no carboxylated beads were used for additionally binding inhibitors and removing precipitates. Thus, PAA alone was shown to be more effective than the combination of PVP and carboxylated beads. However, to additionally clear the lysate from precipitates can be advantageous and thus, is preferred. Furthermore, the results obtained with buffer 2 (B2) show that also the polyanionic support alone is efficient in depleting PCR inhibitors so that the analytical method can be performed. However, the combined use with the polymer(s) achieved significantly better results.

### Example 7: Test of different particles

In this experiment it was tested whether also particles which do not have a polyanionic surface achieve a depletion of inhibitors. Therefore, lysis was performed in the presence of silica beads (Mas G Beads, QIAGEN). For comparison, particles having a carboxylated surface (Sera Mag beads) were used. In separate reaction tubes comprising 12.5 µl of Sera Mag Beads, 12.5 µl of Mas G Beads or no beads (control) 10 µm sections from rat liver tissue (PFPE) were lysed in 250 µl of a lysis buffer (0.1 % PVP (MW 10.000), 0.45 % Tween-20, 0.45 % NP40, 1 mM EDTA, 40 mM Tris/Cl, pH 9). The reactions were performed in duplicates. Lysis reactions were incubated for 15 min at 95°C using a thermomixer, followed by about 5 minutes cooling to room temperature. Beads were separated using magnetic force for about 1 minute, and the supernatant was transferred to a new tube. The lysate was analyzed by PCR using ß-actin as target.

For the PCR detection, the following reaction mixture was used:
12.5 µl QF Probe MM
1 µl fwd primer (10 µM)
1 µl rev primer (10 µM)
1.25 µl probe (10 µM)
5 µl lysate
4.25 µl water

The PCR was performed using 95°C for 5 min (initial denaturation), followed by 40 cycles of denaturation at 95°C for 10 seconds, annealing at 55°C for 30 seconds and extension at 72°C for 30 seconds.

The results show that an amplification product was detected only in lysates derived from samples processed in presence of the carboxylated beads. Therefore, inhibitors could not be efficiently depleted with silica particles.

## Claims

1. Method for lysing a fixed biological sample wherein the obtained lysate is suitable for being directly used in a nucleic acid analysis method, comprising:
a) contacting the fixed biological sample with an aqueous lysis composition thereby providing a lysis mixture;
b) heating the lysis mixture at ≥ 85°C to provide a lysate;
wherein inhibitors of the subsequent nucleic acid analysis method are depleted by
i) contacting the fixed biological sample in step a) with at least one compound which prevents or reduces the inhibition of the subsequent nucleic acid analysis method and/or
ii) binding inhibitors to a solid support comprising an anionic surface.

2. The method according to claim 1, wherein the obtained lysate is suitable for being directly used in a nucleic acid amplification method.

3. The method according to claim 1 or 2, wherein the compound which prevents or reduces the inhibition of the subsequent nucleic acid analysis method is a water-soluble polyanionic polymer which comprises carboxylate-containing monomers.

4. The method according to claim 3, wherein the at least one water-soluble polyanionic polymer has one or more of the following characteristics:
i) said polymer comprises as monomers acrylic acid, methacrylic acid and/or maleic acid;
ii) said polymer comprises acrylic acid and maleic acid, preferably consists of acrylic acid and maleic acid;
iii) said polymer comprises acrylic acid, preferably consists of acrylic acid;
iv) said polymer comprises maleic acid, preferably consists of maleic acid;
v) said polymer is selected from polyacrylic acid, polymaleic acid and a co-polymer comprising polyacrylic acid and polymaleic acid; and/or
vi) said polymer has an average molecular weight that is selected from a range of 2,000 Da to 500,00 Da, 10,000 to 450,000 Da, 25,000 to 400,000 Da, 35,000 to 350,000 Da, 50,000 Da to 300,000 Da, 75,000 to 300,000 Da, 100,000 Da to 300.000 Da, 150,000 to 300,000 Da and 200,000 Da to 300,000 Da.

5. The method according to one or more of claims 1 to 4, wherein the compound which prevents or reduces the inhibition of the subsequent nucleic acid analysis method is a binder and/or thickener, preferably is a polymer selected from the group consisting of polyvinylpyrrolidone (PVP), polyoxazoline, polyethylene glycol and polyvinyl alcohol, more preferred polyvinylpyrrolidone.

6. The method according to one or more of claims 1 to 5, wherein the compound which prevents or reduces the inhibition of the subsequent nucleic acid analysis method is comprised in the aqueous lysis composition and preferably is comprised therein in a concentration that is selected from a range of 0.01% (w/v) to 1% (w/v), 0.02% (w/v) to 0.5% (w/v), 0.025% (w/v) to 0.3% (w/v), 0.035% (w/v) to 0.2% (w/v) and 0.04% (w/v) to 0.15% (w/v).

7. The method according to one or more of claims 1 to 6, wherein a solid support having an anionic surface is used to deplete inhibitors and precipitates, if formed, and wherein the solid support comprising the bound inhibitors and precipitates is separated from the remaining lysate thereby providing a cleared lysate.

8. The method according to one or more of claims 1 to 7, wherein the solid support has an anionic surface which binds inhibitors originating from the lysed sample and has one or more of the following characteristics:
- the solid support is selected from the group consisting of particles, plates and other particulate matter;
- the solid support comprises anionic groups on its surface to promote unspecific binding of inhibitors originating from the lysed sample to the solid support,;
- the solid support comprises carboxyl groups on its surface;
- the solid support has magnetic properties; and/or
- the solid support is provided by magnetic particles which comprise carboxyl groups on the surface.

9. The method according to one or more of claims 1 to 8, having one or more, preferably at least two, at least three, more preferred having all of the following features:
i) the aqueous lysis composition comprises at least one detergent, preferably at least one non-ionic detergent;
ii) the aqueous lysis composition comprises at least one non-ionic detergent, wherein the non-ionic detergent is selected from the group consisting of polyoxyethylene fatty alcohol ethers, polyoxyethylene alkylphenyl ethers, polyoxyethylene-polyoxypropylene block copolymers, alkylglucosides and alkylphenol ethoxylates, preferably polyoxyethylene fatty alcohol ethers, polysorbates and/or polyoxyethylene alkyl phenyl ethers;
iii) wherein in step a) the fixed biological sample is contacted with at least one chelating agent; and/or
iv) wherein in step a) the fixed biological sample is contacted with at least one chelating agent that is selected from the group consisting of diethylenetriaminepentaacetic acid (DTPA), ethylenedinitrilotetraacetic acid (EDTA), ethylene glycol tetraacetic acid (EGTA) and N,N-bis(carboxymethyl)glycine (NTA).

10. The method according to one or more of claims 1 to 9, wherein in step a) the fixed sample is contacted with an aqueous lysis composition which comprises
i) at least one polymer which prevents or reduces the inhibition of the subsequent analytical method preferably by unspecific complexing of potential inhibitors;
ii) at least one non-ionic detergent or mixture of non-ionic detergents selected from the group consisting of polyoxyethylene fatty alcohol ethers, polyoxyethylene alkylphenyl ethers, polyoxyethylene-polyoxypropylene block copolymers, alkylglucosides and alkylphenol ethoxylates, preferably polyoxyethylene fatty alcohol ethers, polysorbates and/or polyoxyethylene alkyl phenyl ethers,
iii) at least one buffering agent; and
iv) optionally at least one chelating agent.

11. The method according to claim 10, having one or more, preferably at least two, at least three, at least four, more preferred having all of the following characteristics:
i) the at least one compound which prevents or reduces the inhibition of the subsequent analytical method is comprised in the aqueous lysis composition and preferably is comprised therein in a concentration that is selected from a range of 0.01% (w/v) to 1% (w/v), 0.02% (w/v) to 0.5% (w/v), 0.025% (w/v) to 0.3% (w/v), 0.035% (w/v) to 0.2% (w/v) and 0.04% (w/v) to 0.15% (w/v);
ii) the at least one non-ionic surfactant or the plurality of non-ionic surfactants is/are comprised in the aqueous lysis composition in a concentration selected from a range of 0.05% (v/v) to 5% (v/v), 0.1 % (v/v) to 2% (v/v), 0.2% (v/v) to 1.5% (v/v) and 0.4% (v/v) to 1% (v/v);
iii) the buffering agent is comprised in the lysis composition in a concentration selected from 5mM to 100mM, 7.5mM to 80mM and 10mM to 50 mM;
iv) the buffering agent is selected from Tris or borate;
v) the pH of the aqueous lysis composition is in a range selected from 7 and 11, 7.5 to 10, 7.75 to 9.5 and 8.0 to 9; and/or
vi) the at least one chelating agent is comprised in the lysis composition in a concentration selected from a range of 0,5mM to 5mM, 0.75mM to 2mM and 1mM to 1.5mM.

12. The method according to one or more of claims 1 to 11, wherein the aqueous lysis composition comprises
- a water-soluble polyanionic polymer which preferably is or comprises polyacrylic acid, in a concentration of 0.01% (w/v) to 0.5% (w/v), preferably 0.03% (w/v) to 0,15% (w/v);
- two non-ionic detergents, wherein each non-ionic detergent is comprised in a concentration of 0.2% (v/v) to 0.6% (v/v), preferably 0.4% (v/v) to 0.5% (v/v);
- a buffering agent, preferably TRIS or borate, in a concentration selected from 7.5mM to 50mM and
- optionally EDTA in a concentration ≤ 1.5mM.

13. The method according to one or more of claims 1 to 12, comprising
a) contacting the fixed biological sample with
- an aqueous lysis composition having a pH value that lies in a range of 7.5 to 10, preferably 8 to 9.5, comprising
i) at least one polymer which prevents or reduces the inhibition of the subsequent analytical method in a concentration of 0.01% (w/v) to 0.5% (w/v), preferably 0.03% (w/v) to 0.15% (w/v), preferably comprising a water-soluble polyanionic polymer comprising carboxylate-containing monomers, more preferred comprising polyacrylic acid;
ii) at least one non-ionic detergent or a mixture of non-ionic detergents,
iii) at least one buffering agent; and
iv) optionally at least one chelating agent;
and
- a solid support comprising an anionic surface which binds inhibitors, wherein preferably the solid support comprises carboxyl groups on the surface,
thereby forming a lysis mixture;
b) heating the lysis mixture at a temperature of ≥ 90°C, preferably 95°C and optionally cooling the lysate;
c) separating the solid support from the remaining lysate.

14. The method according to one or more of claims 1 to 13, having one or more, preferably at least two, at least three, more preferred having at least four of the following characteristics:
aa) the fixed biological sample has one or more of the following characteristics:
i) the biological sample was fixed using a cross-linking fixative;
ii) the biological sample was fixed using formaldehyde and/or paraformaldehyde;
iii) the biological sample was fixed using alcohol; and/or
iv) the fixed biological sample is embedded in a embedding material, preferably in paraffin;
bb) lysis of the fixed sample does not involve the use of chaotropic salts and/or proteolytic enzymes;
cc) the fixed sample is embedded in an embedding material and wherein the method does not comprise a separate step for removing the embedding material prior to step a);
dd) wherein in step b), the lysis mixture is heated for at least 5min, at least 7.5min, at least 10min, at least 12.5min, at least 15min, at least 17.5 min, at least 20min, at least 22.5min, at least 25min, at least 27.5min or at least 30min;
ee) wherein in step b) the lysis mixture is heated for a time period selected from 5 to 45min, 7.5 to 42.5min, 10min to 40min, 12.5min to 37.5min and 15min to 35min; and/or
ff) wherein the method comprises using at least an aliquot of the obtained lysate in a nucleic acid analysis method, preferably in an amplification based nucleic acid analysis method.

15. A nucleic acid analysis method, comprising
a) lysing a fixed biological sample according to the method as defined in one or more of claims 1 to 14, and
b) using at least a portion of the obtained lysate in a nucleic acid analysis method.

16. The method according to claim 15, having one or more of the following characteristics:
i) the lysate is cleared between steps a) and b) and a portion of the cleared lysate is used in step b);
ii) no nucleic acid isolation is performed between steps a) and b);
iii) the lysate is processed between step a) and b) by contacting the obtained lysate with at least one enzyme;
iv) the obtained lysate is used to reconstitute a dry composition, preferably a freeze-dried composition, which comprises reagents for performing the nucleic acid analysis method; and/or
v) the nucleic acid analysis method is selected from or involves one or more of an amplification reaction, a polymerase chain reaction (PCR), isothermal amplification, reverse transcription polymerase chain reaction (RT-PCR), reverse transcription amplification, quantitative real time polymerase chain reaction (qPCR), DNA or RNA sequencing, reverse transcription, LAMP (loop mediated isothermal amplification), RPA (recombinase polymerase amplification), tHDA (helicase dependent amplification), NEAR (nicking enzyme amplification reaction), TMA (transcription mediated amplification) and NASBA (nucleic acid sequence based amplification), allele specific polymerase chain reaction, polymerase cycling assembly (PCA), asymmetric polymerase chain reaction, linear after the exponential polymerase chain reaction (LATE-PCR), helicase-dependent amplification (HDA), hot-start polymerase chain reaction, intersequence-specific polymerase chain reaction (ISSR), inverse polymerase chain reaction, ligation mediated polymerase chain reaction, methylation specific polymerase chain reaction (MSP), multiplex polymerase chain reaction, nested polymerase chain reaction and solid support polymerase chain reaction.
